(19) Europäisches Patentamt
European Patent Office
Office européen des brevets

(11) EP 4 360 690 A2

(12) **EUROPEAN PATENT APPLICATION**

(43) Date of publication:
**01.05.2024 Bulletin 2024/18**

(21) Application number: **24163432.8**

(22) Date of filing: **15.08.2019**

(51) International Patent Classification (IPC):
***A61M 37/00*** *(2006.01)*

(52) Cooperative Patent Classification (CPC):
**A61M 37/0015;** A61M 2037/0023; A61M 2037/0053

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.08.2018 US 201862764685 P**

(62) Document number(s) of the earlier application(s) in accordance with Art. 76 EPC:
**19762024.8 / 3 837 002**

(71) Applicant: **ALLERGAN, INC.**
**Irvine, CA 92612 (US)**

(72) Inventors:
- **LIU, Futian**
  **Lake Forest, 92630 (US)**
- **YU, Xiaojie**
  **Orange, 92867 (US)**
- **STEWARD, Lance E.**
  **Irvine, 92614 (US)**

(74) Representative: **Cohausz & Florack**
**Patent- & Rechtsanwälte**
**Partnerschaftsgesellschaft mbB**
**Bleichstraße 14**
**40211 Düsseldorf (DE)**

Remarks:
This application was filed on 14-03-2024 as a divisional application to the application mentioned under INID code 62.

(54) **MICRONEEDLE ARRAY WITH ACTIVE INGREDIENT**

(57) Microneedle arrays for introducing an active ingredient through a skin surface of a patient can include a base layer, a plurality of microneedles projecting from the base layer, and a shell layer having an active ingredient. Each of the microneedles includes an elongate body having a proximal portion and a distal portion, in which the proximal portion is attached to the base layer. Each of the microneedles can also include at least one dissolvable polymer. The active ingredient is incorporated in the shell layer, which extends around at least a portion of the elongate body.

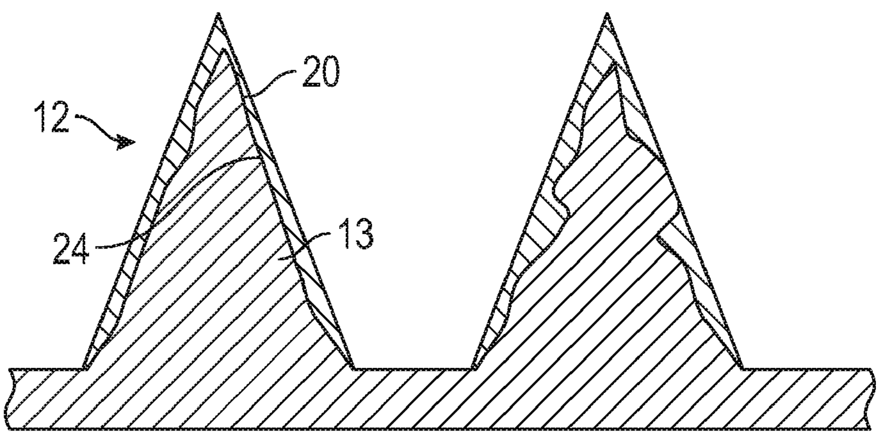


FIG. 1B

EP 4 360 690 A2

## Description

CROSS-REFERENCE TO RELATED APPLICATIONS

This application claims priority to and benefit under 35 U.S.C. § 119(e) from U.S. Provisional Application Serial No. 62/764,685, filed August 15, 2018, the entirety of which is incorporated herein by reference.

BACKGROUND

Field

The present disclosure relates generally to microneedle arrays and methods for producing and using microneedle arrays, and, more particularly, to microneedle arrays and associated methods in which an active ingredient is localized in a shell layer formed on at least a distal end of each microneedle.

Background

Drug delivery into or through the skin can be limited due to the inability of many drugs to penetrate the stratum corneum at therapeutically relevant rates and amounts during topical delivery. One approach that has been taken to improve drug permeability through the skin is to reversibly create a plurality of apertures large enough for drug molecules to pass through. Several techniques have been employed to this end including, for example, chemical permeation enhancement, iontophoresis, electroporation, ultrasonic pressure wave generation, and radiofrequency and/or heat ablation. These approaches can be problematic in some instances, most commonly due to the small aperture size produced. Larger drug molecules, such as biological entities, for example, are frequently too large to traverse the apertures created using these techniques. Because of their large size, biological entities are often administered by hypodermic injection, which can be painful for a patient and undesirable for treating certain skin surface areas.

An alternative approach for creating apertures in the skin utilizes microneedle arrays. Upon application of a microneedle array to a skin surface of a patient, multiple skin penetrations allow drug molecules to pass through. Microneedle-created apertures are dictated by the cross-sectional size of the individual microneedles, which are typically many microns in width. As such, microneedle-created apertures can allow larger drug molecules such as antigens, antibodies and toxins to be introduced through the skin in order to perform a therapeutic function. Because of their small size and limited penetration depth, microneedle arrays do not generally cause significant pain for a patient, unlike hypodermic injections.

Microneedle arrays can be utilized in a number of ways to deliver drug molecules through the skin. In a "poke and patch" approach, a microneedle array is applied to the skin and then removed to create apertures, and a drug or drug patch is then applied topically over the created apertures. Fast healing of the apertures can limit the effectiveness of this approach. In a "poke and flow" approach, hollow microneedles are used to penetrate the skin, and the microneedles are then left in place to serve as a conduit for delivering a liquid drug through the skin.

In a "coat and poke" approach, drug-coated microneedles are used both to create apertures and to deliver drug molecules through the skin. This approach can be appealing for drug-delivery systems in which only a small dose is required. However, the coat and poke approach can also be problematic. For example, the coating of drugs onto the microneedles can be limited in the amount that can be coated on and the coats tend to lack a uniformity among each microneedle. This makes it difficult to ensure that a microneedle array will deliver a desired drug dose. In addition, drug coatings on microneedles can be prone to damage and loss of drug when handled incorrectly. Moreover, drug coated needles face issues of instability as the drug in the coating is more exposed to drug-degrading agents such as heat, light, and oxidants.

In addition, where the active agent is a toxin, for example a neurotoxin, only small nanogram-sized doses may be required, however if the drug is dispersed throughout the entirety of the microneedle array, then the dose might not be effectively delivered in a timely manner. Therefore, in such cases it would be important that the microneedle be configured to provide the entire dose of toxin at a surface of the microneedle where the small amount of toxin is readily accessible to be delivered to a patient while simultaneously avoiding the problems of a "coat and poke" approach to delivery.

SUMMARY

The present application discloses microneedle arrays, and in some embodiments, microneedle arrays that can be used for "poke and release" drug delivery applications, where degradable microneedles loaded with drug molecules are used to penetrate the skin, and after a length of time, the drug-loaded microneedles disengage from their substrate

and are retained in the skin. In accordance with some embodiments, drug molecules or other active ingredients can be utilized more efficiently in this "poke and release" delivery motif through utilizing aspects of the disclosure herein than in conventional microneedle arrays intended for "poke and patch," "coat and poke," or "poke and flow" drug delivery applications.

Some embodiments of the microneedle arrays disclosed herein incorporate an active ingredient in a manner such that essentially the entirety of the active ingredient carried by the array is deliverable to a patient and higher active ingredient loadings are achievable than in conventional microneedle arrays intended for "poke and patch," "coat and poke," or "poke and flow" drug delivery applications. Further, some embodiments of the microneedle arrays discussed herein can be utilized in treating a variety of conditions and can be particularly effective for delivering biological entities, such as toxins, to a patient.

In some embodiments, the microneedles incorporate a toxin in a biodegradable shell layer matrix having the toxin dispersed therein, and further provide a uniformity of drug distribution among each of the microneedles in the array. This shell layer matrix can partially or completely encapsulate a microneedle or alternatively form a "cap" covering at least a portion at the tip of the microneedle. The matrix helps to protect the toxin from environmental degrading agents such as heat, light, and oxidants while also keeping the entirety of the dose at a surface of the needle where it is readily accessible for rapid or controlled delivery. In addition, the shell layer matrix is integrated with the rest of the microneedle making it less prone to damage than a "coat and poke" needle configuration.

In some embodiments, a method is provided for the manufacture of microneedles incorporating a toxin in a biodegradable shell layer. Traditional methods of manufacture have included spray coating or over-molding of drug solutions or matrices onto an already formed needle of a delivery platform; however, in accordance with an aspect of at least some embodiments disclosed herein is the realization that the use of high molecular weight biodegradable polymers, such as hyaluronic acid, can make these methods untenable. In particular, high molecular weight polymers, when added in a sufficient concentration, form viscous solutions that have unfavorable flow characteristics for spray coating. In addition, in accordance with an aspect of at least some embodiments disclosed herein is the realization that spray coating or over-molding onto an already formed needle have difficulties with bonding or integration of the coating onto the outer surface of the microneedles. Further, in accordance with an aspect of at least some embodiments disclosed herein is the realization that it may be difficult to control shell layer thickness and spatial distribution (e.g., partial coverage or full coverage). The methods described herein provide for a way of manufacturing microneedle arrays from viscous polymer solutions while controlling the spatial distribution of a drug-containing outer layer (shell layer) on a microneedle that is integrated with the matrix of the body of the needle. In addition, the method maintains the drug in the outer layer without the drug migrating into the microneedle, such as into a core or central body of the microneedle, during manufacture.

Thus, at least some embodiments disclosed herein can advantageously provide enhanced bonding between a body of a microneedle and a drug-containing outer layer, control the drug-containing outer layer thickness and distribution, and tend to ensure that the amount of drug carried by the microneedles is actually effectively delivered to the patient. This not only improves the effectiveness and reduces the cost of the microneedle array, but also reduces the waste of drugs and cost of the microneedle array.

For example, a reduction in the waste of drugs can be achieved relative to inefficient drug-delivery alternatives in which insufficient bonding causes the drug to be sheared off of or otherwise dislodged from a microneedle's surface during application, which lowers the effectiveness of the array because it precludes proper application and delivery of the drug. Further, a reduction in drug waste can be achieved relative to alternatives having a drug infused throughout an entirety of the microneedle or microneedle tip (i.e., not just coated, but integrated into the body of the microneedle); in such alternatives, if the drug-infused microneedles are not fully injected into or absorbed by the patient, the microneedle array removed from the patient will still have a substantial or meaningful amount of drug that is unused and must be disposed of. In any of these inefficient drug-delivery alternatives, a desired dosage for the patient can only be achieved by loading the microneedle array with an amount of drugs that exceeds the desired drug dosage because the microneedle array cannot effectively administer or deliver substantially the entire amount of drugs borne by the microneedle array. In contrast, the devices and methods disclosed herein can enable a microneedle array to carry a desired dosage and deliver substantially all of the desired dosage to the patient, thereby reducing the cost of the array and drug waste.

In accordance with some embodiments disclosed herein, a microneedle array can be provided in which the active ingredient can be dispersed or dissolved uniformly in the drug-containing layer of each microneedle or in a concentration gradient in the drug-containing shell layer of each microneedle. For example, a concentration gradient of the active ingredient can be provided via layers of different concentrations, either increasing or decreasing concentrations or both increasing and decreasing concentrations, from an inner drug-containing layer to an outer drug-containing layer of each microneedle. In addition, the elongate body of each microneedle and the base layer of the microneedle arrays lack the active ingredient. As such, upon separation of the microneedles from the base layer following application to a skin surface of a patient, none of the active ingredient is lost to waste when the base layer is subsequently removed from the skin surface. Although each microneedle is relatively small and holds only a small amount of active ingredient, therapeutically effective amounts of the active ingredient can be delivered upon collective dissolution or degradation of

the microneedles.

Accordingly, some embodiments of a microneedle array can include a base layer and a plurality of microneedles projecting from the base layer. Each of the microneedles comprises an elongate body having a proximal portion and a distal portion, and the proximal portion is attached to the base layer. The microneedles and the base layer can be dissolvable. For example, the microneedles and the base layer can comprise at least one dissolvable polymer. An active ingredient can be incorporated, dispersed, or dissolved in a polymer matrix that forms an outer layer integrated with at least a portion of the elongate body of each microneedle, with the active ingredient being present only in outer shell layer. The active ingredient can be disposed uniformly or in a gradient fashion in the outer layer.

In some embodiments, the active ingredient present in the microneedle arrays comprises drug molecules or biomolecules (i.e., biological entities). In some embodiments, the active ingredient comprises an antigen, antibody, or toxin. In still some embodiments, the active ingredient is a neurotoxin such as a botulinum toxin, for example. Botulinum toxin of types A, B, C, D and/or E can be present in the microneedle arrays. In some embodiments, the botulinum toxin is selected from the group consisting of Botulinum toxin serotype A (BoNT/A), Botulinum toxin serotype B (BoNT/B), Botulinum toxin serotype C1 (BoNT/C1), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), Botulinum toxin serotype J (BoNT/J), and mosaic Botulinum toxins and/or variants thereof. Examples of mosaic toxins include BoNT/DC, BoNT/CD, and BoNT/FA. In some embodiments, the botulinum toxin can be a sub-type of any of the foregoing botulinum toxins.

In some embodiments, the at least one dissolvable polymer comprises hyaluronic acid, crosslinked hyaluronic acid, hydrophobically modified hyaluronic acid, or any combination thereof.

In some embodiments, a microneedle array is provided comprising a base layer; a plurality of microneedles projecting from the base layer, each of the microneedles and the base layer being a single, structurally continuous component comprised of a first hyaluronic acid polymer matrix, each of the plurality of microneedles being an elongate body having a proximal portion continuous with and adjacent to the base layer, the elongate body generally tapering from the proximal portion toward a distal portion thereof and defining an irregular or random body geometry; and drug-carrying shell layers at least partially encapsulating and flowed around the elongate bodies of the plurality of microneedles to define an irregular or random surface boundary against the irregular body geometries thereof, the drug-carrying shell layers each having a predefined outer profile that varies from a respective irregular body geometry of a respective elongate body to permit the array to have a consistent microneedle profile, the drug-carrying shell layers comprising a neurotoxin dispersed in a second hyaluronic acid polymer matrix.

In some embodiments, a method for forming a microneedle array comprising drug-carrying shell layers or a neurotoxin-containing outer layer around each of the elongate bodies is provided. In some embodiments, the method comprises providing a microneedle array mold comprising a plurality of elongate wells; providing a first hyaluronic acid polymer solution comprising a neurotoxin and about 1 wt. % to about 40 wt. % of a hyaluronic acid; dispensing the first hyaluronic acid polymer solution into a lower portion of each of the elongate wells providing a second hyaluronic acid polymer solution comprising about 25 wt. % to about 50 wt. % of a hyaluronic acid, wherein the viscosity of the second hyaluronic acid polymer solution is greater than the viscosity of the first hyaluronic acid polymer solution; after dispensing the first hyaluronic acid polymer solution, dispensing the second hyaluronic acid polymer solution into each of the elongate wells, the greater viscosity of the second hyaluronic acid polymer solution causing displacement of at least a portion of the first hyaluronic acid polymer solution from the lower portion of each of the elongate wells to flow around the second hyaluronic acid polymer solution thereby forming a neurotoxin-containing outer layer; and drying the first and second hyaluronic acid polymer solutions in the mold to form a microneedle array comprising a base layer having a plurality of microneedles projecting therefrom, each microneedle comprising an elongate body and the neurotoxin-containing outer layer. In some embodiments, after dispensing the second hyaluronic acid polymer solution, the resulting mold assembly (i.e., solutions cast in the mold) can be subjected to compression to assist with and accelerate the displacement of the first hyaluronic acid polymer solution and to cause the two polymer solutions to integrate along an interface whereby the neurotoxin-containing outer layer and the base layer become integrally formed.

Depending on the nature of the hyaluronic acid used (e.g., molecular weight concentration, etc.), the first hyaluronic acid polymer solution and the second hyaluronic acid polymer solution can be rather viscous. Accordingly, in some embodiments, a casting process can be employed to promote fluid dispensation into a desired location within the mold. The casting process can aid dispensation of the first fluid into the lower portion or bottom of each elongate well and promote formation of the resulting microneedles. In some embodiments, the casting process can include applying a pressure (compression), applying a vacuum during dispensation of a solution, centrifugation, shaking, and/or vibrating. In some embodiments, the lower portion of each of the elongate wells can be filled with the first hyaluronic acid polymer solution by depositing, moving, or casting the first hyaluronic acid polymer solution into the lower portion of each of the elongate wells. Likewise, the remainder of the elongate wells can be overfilled with the second hyaluronic acid polymer solution by depositing, moving, or casting. In some embodiments, the second hyaluronic acid polymer solution can be cast above the first hyaluronic acid polymer solution into the mold and/or within each of the elongate wells.

Some embodiments of methods for treating a patient using a microneedle array are also disclosed herein. The methods can involve "poke and release" delivery of an active ingredient through a skin surface of a patient. More specifically, such methods comprise providing a microneedle array described herein, and applying the microneedle array to a skin surface of a patient thereby penetrating the skin surface and embedding the plurality of microneedles in the skin. The microneedle arrays comprise a base layer, a plurality of microneedles projecting from the base layer, and an active ingredient. Each of the microneedles can comprise an elongate body having a proximal portion and a distal portion, and the proximal portion is structurally continuous with the base layer. The microneedles and the base layer comprise at least one dissolvable polymer. An active ingredient is incorporated in the elongate body of each microneedle, with the active ingredient being present only in the distal portion of each elongate body and at least internally within the distal portion.

Upon becoming embedded in the skin surface of the patient, the at least one dissolvable polymer can dissolve or degrade over time under physiological conditions to release the active ingredient to the patient.

After the at least one dissolvable polymer dissolves or degrades, thereby releasing the microneedles from the base layer, the base layer can be removed from the skin surface of the patient. The microneedles and their incorporated active ingredient can thereafter remain within the patient to provide the desired effect.

Additional features and advantages of the subject technology will be set forth in the description below, and in part will be apparent from the description, or may be learned by practice of the subject technology. The advantages of the subject technology will be realized and attained by the structure particularly pointed out in the written description and embodiments hereof as well as the appended drawings.

It is to be understood that both the foregoing general description and the following detailed description are exemplary and explanatory and are intended to provide further explanation of the subject technology.

## BRIEF DESCRIPTION OF THE DRAWINGS

Various features of illustrative embodiments of the present disclosure are described below with reference to the drawings. The illustrated embodiments are intended to illustrate, but not to limit, the present disclosure. The drawings contain the following figures

Figure 1A provides a side-view illustration of a microneedle array, according to some embodiments.

Figure 1B provides a side cross-sectional view of a microneedle of the array shown in Figure 1A, according to some embodiments.

Figure 2A provides a side-view illustration of another microneedle array, according to some embodiments.

Figure 2B provides a side cross-sectional view of a microneedle of the array shown in Figure 2A, according to some embodiments.

Figure 3 provides a side cross-sectional view of a microneedle array configuration that would be expected by a dipping or spray-coating method.

Figure 4 provides a top-view illustration of a microneedle array, according to some embodiments.

Figures 5A-5D show an illustrative process schematic, as observed from a side view, through which a microneedle array is fabricated, according to some embodiments.

Figures 6A-6C show an illustrative schematic demonstrating how a microneedle array of the present disclosure is used to treat a patient, according to some embodiments.

Figure 7 shows a scanning electron microscope (SEM) image of a microneedle array, according to some embodiments.

Figure 8 shows an x-ray micro computerized tomography (CT) image of a microneedle array, according to some embodiments.

Figure 9 shows an image of a microneedle array having an outer shell layer along a distal portion of the microneedles carrying trypan blue, according to some embodiments.

Figure 10 shows a plot of force response versus probe travelling distance for a hyaluronic acid microneedle array in an embodiment disclosed herein in comparison to a commercially available microneedle array obtained from Shiseido, according to some embodiments.

Figure 11A shows a photograph of a skin sample treated with a dye-labeled microneedle array described herein, according to some embodiments.

Figure 11B shows a micrograph of an exemplary microneedle array taken prior to penetration of a skin sample, according to some embodiments.

Figure 11C shows a micrograph of the microneedle array of Figure 9B taken after 5 minutes of penetration of a skin sample, according to some embodiments.

Figure 12 shows the results of a study involving immunoglobulin G (IgG) dermal permeation through human cadaver skin, according to some embodiments.

Figure 13 A shows a micrograph image of a microneedle array having trypan blue dye loaded layer on the microneedles.
Figure 13B shows a cross-sectional micrograph image of a microneedle array having a trypan blue dye loaded layer on the microneedles.
Figure 13C shows a micrograph image of a second microneedle array having a trypan blue dye loaded layer on the microneedles.
Figures 14A-14C show the results of a Rat Digit Abduction Score (DAS) assay for toxin-loaded microneedle array patches described herein.

## DETAILED DESCRIPTION

In the following detailed description, numerous specific details are set forth to provide a full understanding of the subject technology. It should be understood that the subject technology may be practiced without some of these specific details. In other instances, well-known structures and techniques have not been shown in detail so as not to obscure the subject technology. While the present description sets forth specific details of various embodiments, it will be appreciated that the description is illustrative only and should not be construed in any way as limiting. Furthermore, various applications of such embodiments and modifications thereto, which may occur to those who are skilled in the art, are also encompassed by the general concepts described herein.

The present disclosure addresses several challenges associated with conventional microneedle arrays intended for use in "poke and patch," "coat and poke," or "poke and flow" drug delivery applications. Specifically, some embodiments of the microneedle arrays can provide localized incorporation of an active ingredient within a drug-carrying shell layer formed from a polymer matrix on each of the microneedles. These features allow the active ingredient to be used more efficiently, while simultaneously averting the issues associated with common microneedle coating approaches. Further advantageously, at least some embodiments of the microneedle arrays can avoid waste disposal issues for potentially hazardous active ingredients, such as neurotoxins.

In addition, the present disclosure relates to various devices and methods, features of which can also incorporate aspects of copending U.S. Patent Application No. 15/932,365, filed February 16, 2018, the entirety of which is incorporated herein by reference.

In order to illustrate some aspects of the present disclosure, a microneedle array consistent with some embodiments will now be described in further detail. Figures 1A-2B provide side-view illustrations of a microneedle array 10, which contains a plurality of microneedles 12 projecting from a base layer 14. The principal difference between the array illustrated in Figure 1A and the array illustrated in Figure 2A is the extent of coverage of a drug-carrying layer, capsule, or shell of each of the microneedles. However, for simplicity and brevity, various aspects of the arrays can be implemented in either or other arrays. Thus, although the present disclosure may refer to certain features with reference to one of the figures, the discussion of that feature can apply to other aspects of the disclosure as well.

Referring generally to Figures 1A-2B, each of the microneedles 12 can comprise an elongate body 13 that a single, structurally continuous component with the base layer 14. In accordance with at least some embodiments, the structural continuity can be achieved by applying a polymer to a mold and causing the polymer to flow and cure into a shape having both the base layer 14 and the plurality of microneedles 12 extending from the base layer 14.

The elongate bodies 13 of each of the microneedles 12 can includes a distal portion 16 and a proximal portion 18. The proximal portion 18 is structurally continuous with the base layer 14, and the distal portion 16 is spaced apart from the base layer 14 via proximal portion 18. For example, as illustrated, at least a portion of the microneedles 12 can be formed with the base layer 14 as a single, unitary component or part. In some embodiments, at least a portion of the microneedles 12 can be formed during the same step that is used to form the base layer 14, as discussed further herein. The relative lengths of the proximal portion 18 and the distal portion 16 can vary over a wide range, and the particular disposition shown in Figures 1A-2B should be considered illustrative and non-limiting.

According to some embodiments of the present disclosure, an active or first ingredient can be carried by the microneedles 12 only in a drug-carrying layer, capsule, or shell on each of the microneedles. For example, referring to Figures 1A-2B, the elongate bodies 13 of the microneedles 12 comprise a drug-carrying shell layer 20, 22. In the embodiment shown in Figures 1A and 1B, the drug-carrying shell layer 20 can completely or almost completely encapsulate or cover the elongate body 13 of the microneedles 12. However, the shell layer can encapsulate or cover only a portion of the elongate bodies 13 of the microneedles 12.

For example, in the embodiment shown in Figures 2A-2B, the elongate bodies 13 of the microneedles 12 comprise a drug-carrying shell layer 22 that only partially covers the surface of the elongate body 13, such as along a distal end portion thereof (although the shell layer 22 can also surround only a proximal end portion thereof while leaving a distal end portion of the elongate body 13 exposed). In this configuration, the drug-carrying shell layer 22 forms a "cap" on the tip of the elongate body 13 that surrounds an upper portion of the elongate body 13.

As such, in at least some embodiments, the active or first ingredient of the drug-carrying shell layer 20, 22 can

be spaced apart from and not present in the base layer 14. Further, as discussed herein, the elongate body 13 can also be devoid of or lack the active or first ingredient. The active or first ingredient can be disposed uniformly or in a gradient fashion in the drug-carrying layer 20, 22. For example, in some embodiments, the gradient distribution of the active ingredient can be created by sequentially depositing aliquots of differing active ingredient concentrations to form the shell layers 20, 22 of the microneedles 12. Further, in at least some embodiments, the drug-carrying shell layer can comprise one or more different active ingredients that form part of the shell layer.

The elongate body 13 of the microneedles 12 and the drug-carrying shell layers 20, 22 are further defined by an interface 24 between the polymer matrices. This interface can comprise intermolecular bonds between the elongate body polymer matrix and that of the drug-carrying shell layers 20, 22. In addition, the elongate body 13 generally tapers from the proximal portion 18 toward the distal portion 16 and can have an irregular or random body geometry. That is, the body geometry of the elongate body 13 can show a variance or randomness in shape or form among the microneedles 12. This variance or randomness in shape or form can be visually detected by comparing microneedles 12 of the array, for example, using any of a variety of imaging apparatuses. In accordance with some embodiments, the variance or randomness in shape or form refers to irregular body geometry of the elongate bodies 13 that is generally not shaped or constricted by a mold wall or surface when being formed, although some portions of the material of the elongate bodies 13 may contact the mold wall during formation. The irregular geometry along an interface can be further understood when contrasting with the expected shape from a dipping or spray-coating method. With further reference to Figure 3, which shows the expected configuration of a microneedle in which an outer layer 30 has been formed on a microneedle 32 by dipping into a solution, suspension, or resin or by spray-coating onto the microneedle 32. The inner microneedle body 32 has a preformed shape resulting in a generally regular body geometry. The coating conforms along an interface to form a regular inner surface layer geometry, however the outer surface of the coating has an irregular geometry as a result to a randomness associated with the spray-coating and/or dipping processes.

Indeed, as described below, the irregular body geometry of the elongate bodies 13 can result in part from the free flow of a more viscous material into a less viscous material and the random interfaces 24 created therebetween. These interfaces 24 are created during a casting process in which a higher viscosity polymer solution applied to form the elongate body 13 displaces all or a portion of a lower viscosity, drug-carrying polymer solution that when cured, forms the shell layers 20, 22 around or encapsulating at least a portion of the elongate bodies 13. The displaced lower viscosity solution is flowed around the elongate bodies 13 of the plurality of microneedles 12 in a random fashion, thus defining an irregular surface boundary or interface 24 against the irregular body geometries of the elongate bodies 13. Accordingly, the existence of voids or capacity within wells of the microneedle mold will tend to permit the material of the elongate bodies 13 to enter the wells of the microneedle mold, and back pressure exerted against the material used for the elongate bodies 13 may tend to displace the material of the shell layers 20, 22 already deposited into the wells. Further, the shape of the elongate bodies 13 is not predetermined, but is instead randomly developed as the process is performed.

In contrast, the drug-carrying shell layers 20, 22 each have a predefined outer profile (e.g., shape, size, and/or form). This predefined outer profile corresponds to an interior surface of elongate wells in a microneedle array mold. Accordingly, although the inner elongate bodies 13 exhibit an irregular body geometry, the drug-carrying shell layers 20, 22 will define a predetermined profile that is determined by the shape of the elongate wells. The predetermined outer profile of the shell layers allow the microneedle array to have a consistent microneedle profile. The elongate wells of the mold can have a single, constant well profile (e.g., shape, size, and/or form) exhibited in all wells or have wells of varying profiles (e.g., shapes, sizes, and/or forms). Indeed, although the outer profile of the shell layers 20, 22 may be predetermined, the volume of material used for each respective shell layer 20, 22 can vary in response to the shape of the respective elongate body 13 around which the shell layer 20, 22 is formed. As a general principle, the volume of material for the elongate body 13 combined with the volume of material for the shell layer 20, 22 will be equal to the total volume of the well of the mold. However, the relative distribution of volumes of material can vary from microneedle to microneedle, as discussed herein.

Thus, in some embodiments, the disposition, size, or shape of the distal and proximal portions 16, 18 of the elongate body 13 of each of the microneedles 12 can vary over a range of relative lengths, even between in an array 10 that has microneedles 12 of a constant size. Preferably, the elongate bodies of the microneedles can provide a "backbone" or "core" structure on which each shell layer can be supported. In general, the elongate body 13 can comprise at least 1% to about 99% of the length of the microneedles 12, and the balance of the length is defined by the shell layer 20, 22. Thus, in general, the shell layer 20, 22 can comprise at least 1% to about 99% of the length of each microneedle 12.

Further, in some embodiments, the proximal portion 18 can comprise between about 1% to about 10%, between about 10% to about 20%, between about 20% to about 30%, between about 30% to about 40%, between about 40% to about 50%, between about 50% to about 60%, between about 60% to about 70%, between about 70% to about 80%, or between about 80% to about 90% of the length of the elongate body 13. In each case, the distal portion 16 fills out the balance of the overall length. Accordingly, in at least some embodiments disclosed herein, the internal interface between the elongate bodies 13 and the shell layers 20, 22 can be randomly oriented within each respective microneedle

12; as such, the only preset or predetermined parameter of the microneedles 12 may be their outer size, shape, and/or length, which is based on the internal profile of the wells of the mold.

In some embodiments, each of the microneedles 12 can have a length ranging between about 25 microns and about 3000 microns. In some embodiments, each of the microneedles 12 can have a length ranging between about 25 microns and about 1000 microns. In some embodiments, all of the microneedles 12 can have substantially the same length. Microneedle lengths within the foregoing ranges can be effective for penetrating a skin surface of a patient and delivering an active ingredient to the dermis, as discussed further herein. Delivery of an active ingredient to the dermis can improve skin quality and treat a variety of conditions affecting the skin, both cosmetic and clinical.

In some embodiments, each of the microneedles 12 can have a conical or pyramidal geometry suitable for perforating the skin. The conical or pyramidal geometry has a pitch angle associated therewith, such that the microneedles 12 taper to a point or tip suitable for perforating the skin.

In some embodiments, the microneedles 12 can have a tip width ranging between about 1 microns to about 30 microns. In some embodiments, the microneedles 12 can have a tip width ranging between about 4 microns to about 25 microns. The foregoing microneedle widths can be measured at the distalmost end or point of the microneedle or with respect to the location where the proximal portion 18 projects from the base layer 14, given that the microneedles 12 taper to a point in some embodiments. Microneedle tip widths within the foregoing size ranges can generate apertures of suitable size in the skin to deliver active ingredients varying over a wide size range, including biomolecules.

As indicated above, the number, dimensionality, length, width and geometry of the microneedles 12 of the microneedle array 10 is not considered to be particularly limited. Similarly, in some embodiments, a density of the microneedles 12 of the microneedle array 10 can range between about 5 microneedles/$cm^2$ to about 1000 microneedles/$cm^2$ or more.

According to some embodiments of the present disclosure, the microneedles 12 and the base layer 14 can be formed from a dissolvable polymer, such that the microneedles 12 are contiguous with the base layer 14 and project therefrom. Thus, in some embodiments, there is no structural discontinuity between the microneedles 12 and the base layer 14. In some embodiments, the dissolvable polymer of the microneedles 12 blends into the dissolvable polymer of the base layer 14.

As indicated above, the elongate bodies 13 of the microneedles 12 and the base layer 14 can lack the active ingredient of the drug-carrying shell layer 20, 22. In some embodiments, the base layer 14 can optionally comprise the same and/or different dissolvable polymer(s) that are present in microneedles 12. For example, in at least some embodiments, the array 10 can be formed by depositing a first material having a low viscosity into a mold, thereafter depositing a second material having a higher viscosity than the first material into the mold, and finally depositing a third material that overlies the second material. The first material can form the shell layers, the second material can form the elongate bodies, and the third material can form the base layer of the array. The second and third materials can be the same or different. Further, and at least some embodiments, the second and third materials can comprise additional materials or drugs, as desired.

In some embodiments, the microneedles 12 can comprise a first dissolvable polymer, such that the distal portion 16 and the proximal portion 18 both comprise the first dissolvable polymer. In some embodiments, the active ingredient can be incorporated only within a matrix of the first dissolvable polymer of the drug-carrying shell layer 20, 22. In some embodiments, the base layer 14 can also comprise the first dissolvable polymer. Alternately, the base layer 14 can comprise a second dissolvable polymer that differs from the first dissolvable polymer comprising the microneedles 12.

In some embodiments, the microneedles 12 and/or the base layer 14 can each comprise one, two, three, or more polymers or layers, which can comprise dissolvable polymers. For example, the microneedles 12 can comprise a first dissolvable polymer and a second dissolvable polymer, such that distal portion 16 comprises the first dissolvable polymer and the proximal portion 18 comprises the second dissolvable polymer. Accordingly, in such embodiments, the active ingredient can be incorporated in distal portion 16 within a matrix of the first dissolvable polymer. Further, in some embodiments, there may not be substantially any active ingredient present in the second dissolvable polymer within the distal or proximal portions 16, 18 of the elongate body 13. In some embodiments, the base layer 14 can comprise the first dissolvable polymer and/or the second dissolvable polymer. In some embodiments, the base layer 14 comprises only the second dissolvable polymer. Alternately, the base layer 14 can comprise a third dissolvable polymer that differs from the first dissolvable polymer and/or the second dissolvable polymer of the microneedles 12. The particular combination of dissolvable polymers can be chosen to tailor the microneedle properties for a desired application, such as to provide a desired release profile, blending profile, and/or mechanical strength, for example. For example, a faster-degrading dissolvable polymer can be present in the proximal portion of the microneedles 12 to release the distal portion of the microneedles 12 from the base layer 14 to ensure that when the array 10 is removed, the active ingredient in the shell layers of the microneedles 12 remains in the patient.

In some embodiments, the elongate body 13 contains a different concentration of the same active agent or a different active agent than the drug-carrying shell layer 20, 22 and is configured to dissolve at a slower rate than the drug-carrying shell layer 20, 22. In this case, after dissolution of the drug-carrying shell layer 20, 22, the elongate body

13 of the microneedle remains in the skin, and slowly dissolved to provide extended release and long-lasting effects.

In other embodiments, a microneedle array is provided in which the drug-carrying shell layer 20, 22 dissolves at a slower rate than the elongate body 13, thereby providing a long acting effect. The slower dissolving drug-carrying outer layer can be formed from a neurotoxin-containing polymer solution comprising a high molecular weight (i.e., greater than 3 MDa) hyaluronic acid at a concentration of about 0.1 to about 2 wt. %. The faster dissolving elongate body 13 can be formed from a polymer solution, optionally containing a toxin, comprising a low molecular weight (i.e., 50,000 Da to $1\times10^6$ Da) at a concentration ranging from about 5 wt. % to about 40 wt. %. The exact molecular weights of the hyaluronic acid in the two solutions depends upon the desired dissolution rates of the elongate body 13 and the drug-carrying outer layer 20, 22. The concentration of the hyaluronic acid in the two solutions depends on the desired viscosity of the solutions, however the solution forming the drug-carrying shell layer 20, 22 must have a lower viscosity than that forming the elongate body 13.

Optionally, both the microneedles 12 and the base layer 14 comprise the same type of dissolvable polymer. By having the same dissolvable polymer present in both the microneedles 12 and the base layer 14, potential incompatibilities between dissolvable polymers having different properties can be averted. For example, by configuring the microneedle array 10 such that the microneedles 12 and the base layer 14 are formed from the same dissolvable polymer, premature release of microneedles 12 through delamination can be avoided or precluded. However, in some embodiments, different dissolvable polymers can be desirable and advantageously used for some applications, including some applications within the context of the present disclosure.

Figure 4 provides a corresponding top view schematic of the microneedle array 10. Although an 8x8 array has been depicted in Figure 4, it is to be recognized that the array dimensionality can be modified to suit the requirements of a particular application. Furthermore, the array dimensionality need not necessarily have the same number of rows and columns, as depicted in Figure 4. In general, the array 10 can comprise any combination of rows and columns that is suitable for a given intended application. Further, the microneedle arrays are not limited to a rectangular configuration, as depicted in Figure 4. Other illustrative microneedle array shapes can include, for example, circular, ovoid, elliptical, crescent or even irregular shaped, whether in a planar or three-dimensional configuration. For example, some embodiments can be used to provide a contour that facilitates use in treatments for the face or other areas of the body.

In some embodiments, the device may include a first region sized and/or shaped to cover a first portion of skin to be treated, and a second region adjacent and connected to the first region, the second region sized and/or shaped to cover a second portion of skin to be treated. In some embodiments, the first region includes first microneedles projecting from the substrate and having a first length, and the second region includes second microneedles projecting from the substrate and having a second length, different from the first length, projecting from the first region and second microneedles having a second height different from the first height, projecting from the second region. In some embodiments, the first length is at least about 1% greater in length than the second length.

For example, in some embodiments, the first length is at least about 5%, at least about 10%, at least about 20%, at least about 30%, at least about 40%, at least about 50%, at least about 60%, at least about 70%, at least about 80%, at least about 90%, at least about 100%, at least about 150%, at least about 200%, at least about 300%, at least about 500%, at least about 800%, or at least about 1000% greater in length than the second length. In some embodiments, the first microneedles may have a length that is at least about 10% to about 200% greater than the length of the second microneedles. For example, the first microneedles have a length that is about 30%, or about 40%, or about 50%, or about 60%, or about 70%, or about 80%, or about 90%, or about 100%, or about 110%, or about 120%, or about 130%, or about 140%, or about 150%, or about 160%, or about 170%, or about 180%, or about 190%, or about 200% or greater than the length of the second microneedles.

In some embodiments, the first array comprises microneedles having a first length and the second array comprises microneedles having a second length different from the first length. In other embodiments, the first array comprises microneedles having a first spacing and the second array comprises microneedles having a second spacing different from the first spacing.

In order for the microneedles 12 to penetrate the skin surface of a patient effectively, sufficient mechanical strength of the at least one dissolvable polymer is desirable. Due to their relatively good mechanical properties, suitable dissolvable polymers for utilization in some embodiments of the present disclosure include, for example, a glycosaminoglycan, a polysaccharide, collagen, elastin, fibroin, starch, glucomannan, hyaluronic acid, crosslinked hyaluronic acid, hydrophobically modified hyaluronic acid, or any combination thereof. Other types of dissolvable polymers can also be suitable and can optionally be used alone or in combination with the foregoing dissolvable polymers. Carboxymethylcellulose, carboxyethylcellulose, and polyvinylalcohol, for example, are other types of dissolvable polymers that can be utilized in the present disclosure. In particular embodiments, hyaluronic acid can be blended with any of the foregoing dissolvable polymers.

In some embodiments, the polymer matrices of the base layer 14, the elongate bodies 13 of microneedles 12, and the drug-carrying shell layers 20, 22 each comprise hyaluronic acid, crosslinked hyaluronic acid, hydrophobically modified hyaluronic acid, or any combination thereof. Various properties of the hyaluronic acid, crosslinked hyaluronic

acid, or hydrophobically modified hyaluronic acid can be modulated to adjust the release profile of the active ingredient from the microneedles 12 upon application of the microneedle array 10 to a skin surface of a patient. Other dissolvable polymers can also be blended with hyaluronic acid to further modulate these properties. In addition to their ability to incorporate a variety of active ingredients, hyaluronic acid and modified hyaluronic acids can convey their own beneficial properties to a patient's skin, according to some embodiments.

Hyaluronic acid (HA) is a bio polysaccharide found in vertebrate tissues in the extracellular matrix. The name hyaluronic acid can be regarded as slightly misleading, since the hyaluronic acid rather is a glycosaminoglycan consisting of two monosaccharide units; D-glucuronic acid and N-acetyl-D-glucosamine, connected by $\beta(1 \rightarrow 4)$ and $\beta(1 \rightarrow 3)$ glucosidic bonds as shown in the structure:

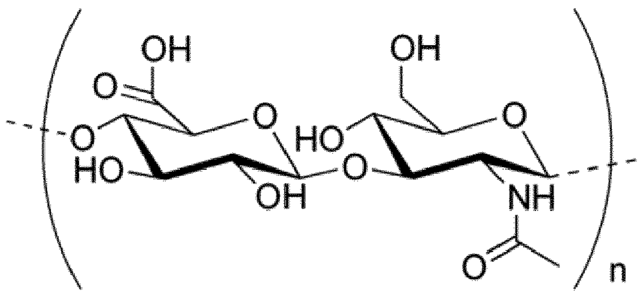

At physiological conditions HA acts like a salt, i.e., sodium hyaluronan. The strongest acidic group, the carboxylic group, in HA has a pKa value of 2.87. This means that the carboxylic group will be deprotonated under physiological conditions. The negative charge of the molecule will interact with positively charged ions and molecules, principally $Na^+$, within the body. Due to this polyanionic charge and because the HA molecule is not branched, the molecule has a rigid and extended conformation. However, in solutions the HA molecule tends to be in a random coil conformation and this is the conformation that has been found when extracting HA from tissues.

Solid HA can dissolve or degrade under physiological conditions or in an aqueous medium. The aqueous medium can be phosphate buffered saline (PBS) or a histidine buffer. The PBS or histidine buffer can have a pH in the range of about 4.0 to about 10.0, about 4.5 to about 9.5, about 5 to about 8, about 5.5 to about 7.5, about 6 to about 8, about 6.5 to about 7.5, about 6.8 to about 7.8, or about 7.0 to about 7.8. HA further dissolves in pH 7.4 PBS or histidine at a temperature in the range of about 10 °C to about 50 °C, about 15 °C to about 45 °C, about 20 °C to about 40 °C, about 25 °C to about 45 °C, about 30 °C to about 40 °C, about 35 °C to about 45 °C, or about 35 °C to about 40 °C.

HA has an exceptional ability to bind water molecules, where the random coils contain almost 99% bound water. Even at highly diluted solutions the extended molecules get tangled together giving the solution great elasticity and viscosity. The elastic and viscous properties of a HA solutions vary depending on the average molecular weight, concentration and the conformation of the polymer. The conformation can differ at different conditions such as pH and temperature. This means that the properties, including viscosity, of the polymer are dependent on the conditions in solution.

The viscosity of a fluid is a measurement of the internal resistance that arises in a flux, which is defined as shear stress ($\tau$) over rate of shear (g). The relationship is here given by Newton's equation:

$$\eta_{rel} = \frac{\eta_x}{\eta_0}$$

Newton's Equation

A fluid can respond to shear stress in different ways. Based on the behavior of a fluid it can be classified as Newtonian or non-Newtonian. For a Newtonian fluid, there is a direct correlation between shear stress and deformation. This linear dependence means that viscosity remains constant regardless of the shear stress. The response is the opposite for a non-Newtonian fluid, as it can typically behave shear thinning or thickening. In this context, viscosity can either decrease or increase under stress. HA has a non-Newtonian fluid behavior, meaning that under stress, it exhibits shear-thinning behavior.

Highly viscous liquids flow more slowly due to higher resistance compared to low viscous liquids. The size of a molecule has a large effect on the viscosity. Therefore, polymers highly affect the viscosity, even at very low concentrations. Outstretched macromolecules cause higher viscosity compared to coiled up molecules.

When working with HA solutions, it is useful to consider the intrinsic viscosity. Intrinsic viscosity [η] is a parameter used to describe the hydrodynamic properties of a macromolecule. It is defined as 1/concentration; this can be compared with viscosity that has the unit *Pa*·s. To determine the [η], one can measure the inherent or the specific viscosity. [η] on the other hand is estimated through extrapolation of these values to infinite dilution, The relationship of intrinsic viscosity [η] to molecular weight (*M*) of HA is given in the Mark-Houwink formula, $M=k\cdot\eta^{\alpha}$, where the values of *k* and $\alpha$ depend on the nature of the polymer in question.

Thus, the viscosity of a hyaluronic acid polymer solution can be controlled by varying the average molecular weight and the concentration of the hyaluronic acid in the solution. For example, a high viscosity can be achieved by using a higher molecular weight (e.g., 6 MDa) HA with only a 5-10% by weight solution. In contrast, for a HA having a molecular weight of 150 kDa, a larger concentration of about 30 weight % is needed in order to achieve a high viscosity solution. In turn, these parameters likewise affect the density of the polymer solution, and upon drying will affect the amount of polymer present in a matrix and the specific weight (i.e., the weight per unit volume) of a polymer matrix.

In some embodiments, the hyaluronic acid, crosslinked hyaluronic acid, or hydrophobically modified hyaluronic acid can have a molecular weight ranging between about 10 kDa and about 6000 kDa. In some embodiments, the at least one dissolvable polymer comprises un-crosslinked hyaluronic acid, crosslinked hyaluronic acid, or hydrophobically modified hyaluronic acid having a molecular weight ranging between about 100 kDa and about 6000 kDa. In some embodiments, the hyaluronic acid is a hyaluronic acid salt selected from sodium, potassium, ammonium, magnesium, and calcium.

In some embodiments, crosslinked hyaluronic acid can have a storage modulus (G') of about 100 Pa to about 3000 Pa. Suitable crosslinking agents for forming crosslinked hyaluronic acid include, for example, epoxy crosslinking agents such as 1,4-butanediol diglycidyl ether (BDDE), divinyl sulfone (DVS), or molecules containing at least two amine groups. In some embodiments, crosslinked hyaluronic acid can be formed via a thiol-Michael addition reaction. For example, thiolated hyaluronic acid can be crosslinked with maleimide-, vinyl sulfone-, or (meth)acrylate-modified hyaluronic acid. Polymer crosslinking in the presence of the active ingredient results in a well-mixed first fluid for use in some embodiments disclosed herein.

In some embodiments, hydrophobically modified hyaluronic acid can include hyaluronic acid that has been functionalized with alkyl or acyl groups, particularly alkyl groups. Alkyl groups suitable for forming hydrophobically modified hyaluronic acid include, for example, ethyl, propyl, benzyl and octyl groups, which can be linear or branched.

In some embodiments, hydrophobically modified hyaluronic acid can be swollen in the presence of phosphate buffered saline (PBS) or dimethyl sulfoxide (DMSO). Other hyaluronic acid compounds can be swollen similarly for use in various embodiments.

Dissolvable polymers having sufficient mechanical strength for forming a microneedle array can produce very viscous fluids upon being disposed in or mixed with a solvent. The high fluid viscosity can result in difficult introduction to a microneedle array mold for forming the microneedle arrays of the present disclosure. Suitable methods for addressing excessive fluid viscosity when forming a microneedle array are discussed in more detail hereinbelow. As such, microneedle arrays of the present disclosure can incorporate a wide range of dissolvable polymers. In addition, the present disclosure is compatible with a range of active materials, illustrative examples of which are discussed hereinafter.

As used herein, the term "active ingredient" refers to any substance which has a therapeutically desired effect when administered to a patient through the skin. In some embodiments, the active ingredient in the microneedle array can differ from the dissolvable polymer(s) present in the microneedle arrays. In particular embodiments, active materials suitable for the microneedle arrays of the present disclosure include antigens, antibodies, and toxins. Since the microneedle arrays of the present disclosure lack an active material in the base layer, potential biohazardous waste disposal issues can be avoided when incorporating these biological entities in the microneedle arrays of the present disclosure.

Neurotoxins, particularly a botulinum toxin, can be especially desirable for at least some embodiments of the microneedle arrays disclosed herein. Any of botulinium toxin types A, B, C, D, E or any combination thereof can be carried by the microneedle arrays of the present disclosure. In some embodiments, the botulinum toxin is selected from the group consisting of Botulinum toxin serotype A (BoNT/A), Botulinum toxin serotype B (BoNT/B), Botulinum toxin serotype C1 (BoNT/C1), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), Botulinum toxin serotype J (BoNT/J), and mosaic Botulinum toxins and/or variants thereof. Examples of mosaic toxins include BoNT/DC, BoNT/CD, and BoNT/FA. In some embodiments, the botulinum toxin can be a sub-type of any of the foregoing botulinum toxins.

The amount of active ingredient integrated into the microneedle array can vary, and may depend on several factors including but not limited to the type of active ingredient, the intended area of application, the type of treatment that is being conducted, the dose to be delivered, and the efficiency of delivering the active ingredient to the host from the device. In some embodiments, the microneedle array can comprise from 0.001% to about 15%, about 0.001% to about 10%, about 0.001% to about 3%, about 0.001% to about 1%, about 0.001% to about 0.5%, or 0.001% to about

0.1% by weight of the entire microneedle array of an active ingredient.

In some embodiments, the active ingredient is present only a distal portion of each of the microneedles of the microneedle array. In such a configuration, the distal portion of each of the microneedles can comprise from about 0.001% to about 15%, about 0.001% to about 10%, about 0.001% to about 3%, about 0.001% to about 1%, about 0.001% to about 0.5%, or 0.001% to about 0.1% by weight of the distal portion of an active ingredient. In some embodiments, the active ingredient present only a distal portion of each of the microneedles of the microneedle array is a neurotoxin. In some embodiments, the distal portion of each of the microneedles can comprise from about 0.000001% to about 0.015%, about 0.000001% to about 0.001%, about 0.000001% to about 0.0001%, about 0.000001% to about0.0000 1%, by weight of the distal portion of a neurotoxin. In some embodiments, the active ingredient is a biologic. In some embodiments, the biologic is a vaccine, a hormone, or a therapeutic peptide. In some embodiments, the distal portion of each of the microneedles of the microneedle array can comprise about 0.000001% to about 1% by weight of the biologic.

In some embodiments, the microneedle array can be configured into a patch for delivery of the active ingredient. In some embodiments, the patch is configured to deliver an effective amount of the active ingredient.

The dosage delivery can be measured in Units (U) per square centimeter. In toxicology, units of a given toxin can be determined by the LD50 of the toxin, the dose required to be lethal to half of a test population. In some embodiments, the patch is configured to deliver a toxin in the amount of about 0.01 to about 100 U/$cm^2$, about 0.05 to about 95 U/$cm^2$, about 0.10 to about 90 U/$cm^2$, about 0.20 to about 85 U/$cm^2$, about 0.25 to about 80 U/$cm^2$, about 0.50 to about 75 U/$cm^2$, about 0.75 to about 70 U/$cm^2$, about 1.0 to about 65 U/$cm^2$, about 2.0 to about 60 U/$cm^2$, about 3.0 to about 55 U/$cm^2$, about 4.0 to about 50 U/$cm^2$, about 5.0 to about 45 U/$cm^2$, about 5.0 to about 40 U/$cm^2$, about 5.0 to about 35 U/$cm^2$, about 5.0 to about 30 U/$cm^2$, about 5.0 to about 25 U/$cm^2$, about 0.01 to about 20 U/$cm^2$, about 0.01 to about 15 U/$cm^2$, about 0.01 to about 10 U/$cm^2$, about 0.01 to about 5 U/$cm^2$, about 0.10 to about 15 U/$cm^2$, about 0.10 to about 10 U/$cm^2$, about 0.05 to about 10 U/$cm^2$, about 0.01 to about 3.0 U/$cm^2$, about 0.10 to about 3.0 U/$cm^2$, or about 0.05 to about 3.0 U/$cm^2$.

In some embodiments, the active ingredient has a loading concentration that exceeds the intended delivery dose to compensate for inefficient delivery. For example, for a composition configured to deliver a dose of 0.1 U/$cm^2$ having a delivery efficiency (percent of total drug contained in composition that is delivered to the patient) of 0.1%, the loading concentration would be about 100 U/$cm^2$. In some embodiments, the loading concentration is in the range of about 0.01 to about 100,000 U/$cm^2$, about 0.10 to about 80,000 U/$cm^2$, about 0.50 to about 50,000 U/$cm^2$, about 1.0 to about 25,000 U/$cm^2$, about 2.0 to about 15,000 U/$cm^2$, about 0.10 to about 20,000 U/$cm^2$, about 0.10 to about 15,000 U/$cm^2$, about 0.10 to about 10,000 U/$cm^2$, about 0.10 to about 8,000 U/$cm^2$, about 0.10 to about 5,000 U/$cm^2$, about 0.10 to about 1,000 U/$cm^2$, about 5.0 to about 1,000 U/$cm^2$, about 5.0 to about 10,000 U/$cm^2$, about 10 to about 100,000 U/$cm^2$, about 10 to about 90,000 U/$cm^2$, about 10 to about 75,000 U/$cm^2$, about 10 to about 50,000 U/$cm^2$, about 10 to about 25,000 U/$cm^2$, about 10 to about 10,000 U/$cm^2$, about 10 to about 1,000 U/$cm^2$, about 10 to about 500 U/$cm^2$, about 10 to about 250 U/$cm^2$, about 10 to about 100 U/$cm^2$, or about 10 to about 50 U/$cm^2$.

Advantages of some embodiments of the microneedle arrays disclosed herein include increasing skin permeability for a variety of biological entities or other active ingredients, permitting slow therapeutic release to be realized while reducing complications from localized or systemic diffusion, providing a large treatment area relative to a single-site injection, and creating less pain for a patient compared to hypodermic administration of an active material. In some embodiments, the microneedle arrays can optionally contain hyaluronic acid with one or more excipients. Hyaluronic acid with adequate molecular weight (e.g., 150 kDa to 6000 kDa) serves as the base material for maintaining microneedle integrity, while excipients such as, for example, sucrose, maltose, polyethylene glycol, or low molecular weight polymers (e.g., hyaluronic acid having a molecular weight <100 kDa), which can dissolve quickly in the skin to promote better delivery of the active ingredient. In some embodiments, the excipient can be a low molecular weight hyaluronic acid having a molecular weight of about 5 kDa, about 10 kDa, about 15 kDa, about 20 kDa, about 25 kDa, about 30 kDa, about 35 kDa, about 40 kDa, about 45 kDa, about 50 kDa, about 55 kDa, about 60 kDa, about 65 kDa, about 70 kDa, about 75 kDa, about 80 kDa, about 85 kDa, about 90 kdA, or about 95 kDa.

Facile methods are also described herein for fabricating the microneedle arrays discussed hereinabove. Figures 5A-5D show an illustrative process, as observed from a side view, through which a microneedle array can be fabricated with drug-carrying shell layers. As shown in Figure 5A, methods for fabricating the microneedle array 110 first include providing the microneedle array mold 120 containing a plurality of elongate wells 122. Each of the elongate wells 122 can contain a lower portion 124 and an upper portion 126.

With reference to Figure 5B, a first fluid, such as a first HA polymer solution, can comprise a hyaluronic acid and an active ingredient, such as a neurotoxin, is then prepared or provided. The first HA polymer solution is then used to partially fill each of the elongate wells 122. This first HA polymer/neurotoxin solution should be of a relatively low viscosity such that it has favorable flow properties and can be displaced as needed. Specifically, the first HA polymer solution fills the lower portion 124 of each of the elongate wells 122, thereby leaving the upper portion 126 unfilled. As shown, at least a portion of the well 122 has capacity to receive an additional fluid, as discussed below. A casting or deposition process can be utilized, as needed, to deposit the first HA polymer solution into the bottom of the elongate

wells 122. Settling can take place during the casting process. In any case, the active ingredient or neurotoxin remains incorporated throughout a matrix of the HA of the first HA polymer solution within the lower portion 124 of each of the elongate wells 122. Suitable methods of casting polymers are described in U.S. Publication No. 2016/0279401, which is herein incorporated by reference in its entirety. Further, U.S. Publication No. 2016/0279401 also discusses additional features of microneedle arrays that can be combined with one or more features of the disclosure herein.

For example, any of the drug molecules or other active ingredients disclosed herein can be homogeneously embedded or incorporated into the polymeric matrix of the drug-carrying shell layer. By forming the microneedles with a drug or other active agent homogeneously incorporated into the polymeric matrix, the release rate of the drug or active agent can be carefully controlled. In addition, incorporation of the drug or active agent may provide the further benefit of uniformity that is not found in needles in which the drug or active agent is coated onto a surface. Further, incorporation can prevent the loss of active agent due to detachment from the surface of the microneedles.

With reference to Figure 5B, a second fluid, such as a second HA polymer solution, is prepared or provided, which lacks the active ingredient of the first fluid or any other active ingredient. In some embodiments, a second HA polymer solution can consist of or consist essentially of a hyaluronic acid in admixture with a solvent. The second HA polymer solution can further comprise additional polymers and excipients that provide mechanical strength and assist in control of dissolution rate. The second HA polymer solution has a relatively high viscosity as compared to the first HA polymer solution. The second fluid is then used to fill the remainder of the elongate wells 122, specifically the upper portion 126 of each of the elongate wells 122. As seen in Figures 5C and 5D, this higher viscosity allows the second HA polymer solution to displace the first HA polymer solution from the lower portion 124 of the elongate wells and to flow around the high viscosity second HA polymer solution and along the interior walls of the elongate wells thereby forming a drug-carrying shell layer around the second HA polymer solution. In addition, the high viscosity is important to ensure that the base layer and the elongate body 13 have a sufficient polymer density and specific weight to provide mechanical strength to the microneedles.

Alternatively, a microneedle array can be manufactured by dispensing the first hyaluronic acid polymer solution over the mold. The higher viscosity hyaluronic acid polymer solution is then cast over the first hyaluronic acid solution, and a pressure or compression is applied above the second hyaluronic acid solution. In a first phase of compression, the less viscous first hyaluronic acid solution will flow into the elongate wells of the mold and then up and around the higher viscosity second hyaluronic acid solution. In a second phase of compression, by further maintaining the pressure for a period of time, hyaluronic acid can diffuse between the two solutions and at an interface formed therebetween. This diffusion creates an integrated hyaluronic acid matrix that binds or fuses the drug-carrying shell layer to the elongate body of the microneedle. Importantly, diffusion of the drug or active ingredient (e.g., a toxin) into the higher viscosity second hyaluronic acid polymer solution does not occur during this process. Thus, the drug-carrying material forming the shell layers and the elongate bodies are diffused, mixed, or fused together via an integrated hyaluronic acid polymer matrix in which the first hyaluronic acid polymer matrix and the second hyaluronic acid polymer matrix overlap or diffuse with each along the irregular surface boundary.

In order to achieve connectivity between the microneedles 112 of the microneedle array 110, the elongate wells 122 can be overfilled with the second HA polymer solution. The overfilled second HA polymer solution can thereby coalesce into a single, continuous layer 130 above elongate wells 122 of the microneedle array mold 120. The second HA polymer solution can be settled through utilization of a second casting or deposition process, as needed, to deposit the second HA polymer solution deeper into elongate wells 122. The settling force can be further conveyed from the second HA polymer solution to the first HA polymer solution to result in further displacement, deposition or filling of the elongate wells 122 and/or densification of the microneedles 112. The continuous layer 130 can thereafter become a base layer 114 upon solidification, where the dissolvable polymer in the second HA polymer solution subsequently defines the base layer 114. Finally, after solidification, the base layer 114 and the remainder of the microneedle array 110 can be released or removed from the microneedle array mold 120.

Once the first HA polymer solution and the second HA polymer solution have been disposed within the microneedle array mold 120, the mold 120 and the microneedle array 110 can be heated to dry the fluids or drive off solvent to form the microneedle array 110, leaving behind dissolved hyaluronic acid polymers and the active ingredient. Alternately, the first and second HA polymer solutions can be evaporated or dried at room temperature to leave behind the dissolved hyaluronic acid polymers and the active ingredient in the form of microneedle array 110. After formation, the microneedle array 110 contains base layer 114 and a plurality of microneedles 112 projecting therefrom with a drug-carrying shell layer 132. Following release from the microneedle array mold 120, the microneedle array 110 is ready for further use.

Referring still to Figure 5D, and with further reference to Figures 1A and 2A, it can be seen that the microneedles 112 and the base layer 114 are structurally continuous with one another or formed as a single, unitary component. For example, the material of the base layer 114 flows continuously into or with each of the microneedles 112, such that there are no structural discontinuities between the base layer 114 and the microneedles 112. Specifically, the proximal portion 116 and the base layer 114 are contiguous with one another. In some embodiments, the proximal portion 116 and the

base layer 114 are substantially the same compositionally. Both the proximal portion 116 and the base layer 114 can lack the active ingredient of the drug-carrying shell layers or other active ingredients, as discussed above in regard to Figures 1A-2B. The drug-carrying shell layers 132 of the microneedles 112, in contrast, can contain the active ingredient incorporated throughout a matrix of the hyaluronic acid. In some embodiments, the active ingredient can be adhered more robustly and uniformly to microneedles 112 than is achieved by coating approaches due to the intimate mixing between the hyaluronic acid polymer and the active ingredient in the first HA polymer solution. In some embodiments, the first HA polymer solution or a plurality of first HA polymer solutions (e.g., first, second, third, or more solutions) can be introduced into mold 120 to introduce a concentration gradient of the active ingredient, dissolution or degradation gradient, or other chemical or mechanical properties in distal portion 118.

The microneedle array molds employed in fabricating some embodiments of the microneedle arrays disclosed herein can have any variety of sizes, shapes, well depths or dimensions, or composition. In some embodiments, the mold can be a silicone mold, which can facilitate the release of the microneedle arrays following their fabrication. Other materials can also facilitate the release of microneedle arrays from a microneedle array mold and can suitably be employed in the disclosure herein. For example, in some embodiments, a microneedle array mold can be silicone-coated or polytetrafluoroethylene-coated to facilitate release of a microneedle array from the mold.

In general, microneedle array molds can contain elongate wells of desired dimensions and number to promote formation of a microneedle array having desired properties. In addition, the microneedle array molds include an area for containing the second HA polymer solution upon overfilling the elongate wells. For example, in some embodiments, a lip can be present around the microneedle array mold to contain or permit pooling of the overfilled quantity of the second HA polymer solution as a continuous layer above the elongate wells. As discussed herein, an overfilled quantity or portion of the second HA polymer solution of the microneedle array mold can be converted into the base layer upon forming the microneedle arrays.

Any suitable technique can be used for introducing the first HA polymer solution and the second HA polymer solution into the elongate wells of the microneedle array molds. Since certain dissolvable polymers, particularly hyaluronic acid or crosslinked hyaluronic acid, can impart significant viscosity to the first HA polymer solution and/or the second HA polymer solution, it can sometimes be difficult to introduce the first HA polymer solution and/or the second HA polymer solution into the elongate wells of the microneedle array molds. Accordingly, in some embodiments of the methods disclosed herein, a casting process can be applied to the first HA polymer solution, the second HA polymer solution or both when they are in contact with the microneedle array mold. In illustrative embodiments, the casting process can utilize a vibrational mechanism or a cast mechanism to promote deeper HA polymer solution penetration into the elongate wells of the microneedle array mold.

In the case of lower viscosity (e.g., less than 5 wt. % of 160 kDA HA) HA polymer solutions, the casting process can utilize centrifugal force or a similar technique to force the first HA polymer solution or the second HA polymer solution deeper into the elongate wells of the microneedle array mold. Other types of casting processes can also be utilized similarly to promote full penetration of the HA polymer solution(s) into the mold.

After introducing the first and second HA polymer solutions into the microneedle array mold, the first and second HA polymer solutions can be heated, dried, and/or evaporated at room temperature to drive off solvent therefrom and result in consolidation of the first polymer and the second polymer within the mold to form the microneedle array. Techniques for heating the mold can include, for example, direct radiant heating, resistive heating, heated air circulation, microwave heating, other suitable techniques, or any combination thereof.

Manufacture of microneedle arrays having a drug containing layer like those illustrated in Figures 1B, 2B, and 5D requires a careful control of the viscosities of the polymer solutions being cast into an array mold. In some embodiments, the layering method of the present disclosure may differ from an over-molding technique. For example, an over-molding technique is effectively the use of layering effects in polymer application techniques. This process is centered around the use of a liquidous resin to add additional layers of shape and structure to an existing component.

In contrast, in at least some embodiments, the arrays and method provided herein have a shell layer that is created by manipulating relative viscosities or densities of polymer casting solutions while both are in the liquid state. Thus, in such embodiments, the drug-containing layer is not being added to an already existed shape. As discussed above, various advantages are now possible using these innovative arrays and methods, including better mechanical stability, reduced drug waste, and reduced cost.

In some embodiments, a method for forming a microneedle array comprises dispensing a first hyaluronic acid polymer solution comprising a neurotoxin and about 1 wt. % to about 40 wt. % of a hyaluronic acid into each of the elongate wells of a microneedle array mold. In some embodiments, the first hyaluronic acid polymer solution comprises about 5 wt. % to about 30 wt. % of a hyaluronic acid. In other embodiments, the first hyaluronic acid polymer solution comprises about 5 wt. % to about 20 wt. % of a hyaluronic acid. In yet other embodiments, the first hyaluronic acid polymer solution comprises about 5 wt. % to about 15 wt. % of a hyaluronic acid. In yet other embodiments, the first hyaluronic acid polymer solution comprises about 1 wt. % to about 10 wt. % of a hyaluronic acid. The dispensing can be performed under vacuum in order to concentrate the first hyaluronic acid polymer solution into the bottom of the

elongate wells. This first hyaluronic acid polymer solution will ultimately form a neurotoxin containing layer or cap on the surface of a microneedle elongate body. The degree to which the neurotoxin containing layer covers the elongate body can be controlled by increasing or decreasing the volume of the first hyaluronic acid polymer solution dispensed into the elongate wells.

After the first hyaluronic acid polymer solution has been dispensed into the elongate wells, a second hyaluronic acid polymer solution comprising about 25 wt. % to about 50 wt. % of a hyaluronic acid is dispensed into the elongate wells of the microneedle array mold. In some embodiments, the second hyaluronic acid polymer solution comprises about 30 wt. % to about 45 wt. % of a hyaluronic acid. In other embodiments, the second hyaluronic acid polymer solution comprises a high molecular weight hyaluronic acid having a molecular weight from about 500 kDa to about 5 MDa in a concentration in the range of about 5 wt. % to about 20 wt. %. The selection of the proper molecular weight and concentration in each of the first and second hyaluronic acid solutions will be apparent to a person of ordinary skill in the art based on the desired compositions of the elongate body and the outer layer.

In accordance with at least some embodiments, the viscosity of the second hyaluronic acid polymer solution is greater than the viscosity of the first hyaluronic acid polymer solution. Upon dispensation of the second hyaluronic acid polymer solution into the elongate wells, this difference in viscosities, which can also be correlated to a difference in densities, causes the second hyaluronic acid polymer solution to displace all or a portion of the first hyaluronic acid polymer solution. The displaced first hyaluronic acid polymer solution flows in an upward direction between the second hyaluronic acid polymer solution and the walls of the elongate wells thereby forming a layer around the second hyaluronic acid polymer solution. The greater viscosity of the second hyaluronic acid polymer solution further prevents the less viscous first hyaluronic acid solution from mixing with the second solution as it is being displaced. Thus, the neurotoxin is maintained in an outer layer.

In some embodiments, the first hyaluronic acid polymer solution and the second hyaluronic acid solution are co-molded. In co-molding, different viscous materials are injected into a mold simultaneously, as opposed to placing one material as an additional layer relative to another. In other words, a sandwich-like structure is created where both materials mold around each other as dissimilar liquids.

The combined solutions in the microneedle array mold can then be subjected to compression. This step helps in forming an integral interface between the first and second solutions prior to drying. After compression and drying, the resulting neurotoxin containing layer and elongate body are structurally integrated with one another, which includes strong intermolecular bonding between polymers. The elongate body, being formed from a higher viscosity solution, can have a greater concentration of polymer in the matrix that forms after drying than the matrix formed in the neurotoxin containing layer. Thus, the elongate body can have a specific gravity, or mass per unit volume, greater than the outer layer. The outer layer will likewise be formed into a hyaluronic acid polymer matrix having the neurotoxin dispersed therein. The dispersion can be uniform. This polymer matrix serves to protect the neurotoxin from degradation agents, forms a strong and stable outer layer with decreased propensity to damage during handling and administration, and further helps control the rate of release of the neurotoxin.

The microneedle arrays of the present disclosure can be used in various treatment methods. In general, the treatment methods can comprise applying a microneedle array of the present disclosure to a skin surface of a patient to embed the plurality of microneedles in the skin surface. Upon becoming embedded in the skin surface, the microneedles can penetrate the epidermis and enter the dermis. In accordance with at least some embodiments, the microneedle array can remain applied to the skin surface for a sufficient length of time for the shell layer to be released from the microneedle. Further, the microneedle array can also remain applied to the skin surface for a sufficient length of time for at least a portion of the active ingredient to be released from the shell layer into the dermis.

Furthermore, in some embodiments, the microneedle array can remain applied to the skin surface until sufficient dissolvable polymer dissolves to affect release of the microneedles from the base layer, thus leaving the microneedles behind in the epidermis or dermis following removal of the base layer from the skin surface. If they are not already completely degraded by the time the microneedle array is removed from the skin surface, the microneedles can break from the base layer at a point of weakness along the microneedle and remain in the skin degrade over time to release their active material to the patient.

Conditions that can be treated with the microneedle arrays of the present disclosure include, but are not limited to, forehead lines, crow's feet, frown lines, finelines, hyperhidrosis, scarring, psoriasis, inflammatory dermatosis, and the like.

For example, Figures 6A-6C show an illustrative schematic demonstrating how a microneedle array of the present disclosure is used to treat a patient. As shown in Figure 6A, a microneedle array 200 can be applied to skin surface 202 of a patient. The skin surface 202 includes epidermis 204 and dermis 206. Upon being applied to the skin surface 202, the microneedles 212 penetrate the epidermis 204, and a drug-carrying shell layer 218 of each of the microneedles 212 at least partially enters the dermis 206. In Figure 6B, the drug-carrying shell layer 218 of each microneedle 212 fully enters the dermis 206. At this juncture, the microneedle array 200 is allowed to remain in place on the skin surface 202 for at least a sufficient length of time for the drug-carrying shell layer 218 to begin dissolution and/or

separate from the microneedle 212 and/or the base layer 214. As depicted in Figure 6C, drug-carrying shell layer 218 (and possibly the microneedles 212) can completely dissolve within the skin surface 202, dermis 206, or epidermis 204 before removal of base layer 214 (and any remaining microneedles 212) therefrom. However, full degradation or dissolution of the polymers is not required. Upon degradation of drug-carrying shell layer 218, active ingredient 220 is released into dermis 206 for performing a therapeutic function.

Examples

**Example 1:** A highly viscous fluid of hyaluronic acid was prepared by hydrating a dry hyaluronic acid fiber having a molecular weight of 500 kDa with phosphate buffered saline. The viscous fluid was transferred to a 1 mL syringe and centrifuged for 10 minutes at 4000 rpm to remove air bubbles. No active ingredient was employed in this example.

After removing air bubbles, 0.20 g of the viscous fluid was placed on a silicone microneedle mold. The applied fluid was cast to a thin film. After casting the fluid, the wet film and the mold were placed in an oven and heated at 40 °C for 2.5 hours. Following heating, a freestanding microneedle array was removed from the mold using a pair of tweezers. The thickness of the base layer was adjusted by casting different quantities of the viscous fluid onto the mold.

Figures 7 and 8 illustrate images of typical microneedles arrays produced using some embodiments of the manufacturing methods herein, including that discussed above with respect to Example 1. Figure 7 shows a scanning electron microscope (SEM) image of the microneedle array, and Figure 8 shows an x-ray micro computerized tomography (CT) image of the microneedle array.

**Example 2:** A microneedle array was prepared in a similar manner to Example 1, except a crosslinked hyaluronic acid was used in place of hyaluronic acid. Specifically, Juvederm Ultra Plus gel was lyophilized and reconstituted to a thick fluid having a hyaluronic acid concentration of 120 mg/mL. A mixture of hyaluronic acid and collagen or hyaluronic acid and fibroins was used similarly. No active ingredient was employed in this example.

**Example 3:** A microneedle array was prepared in a similar manner to Example 1, except a hydrophobically modified hyaluronic acid was used. Specifically, benzylated hyaluronic acid having a degree of benzylation of 80% was mixed with dimethyl sulfoxide to form a viscous paste containing 20 wt. % hyaluronic acid. In this case, drying took place in the oven for 24 hours at 45 °C. No active ingredient was employed in this example.

**Example 4:** In this example, trypan blue (MW = 873) was used as a model active ingredient for formulation with hyaluronic acid in forming a microneedle array. Trypan blue and hyaluronic acid were dissolved in PBS to form a viscous paste. The viscous paste was then cast in the silicone mold. After casting the trypan blue/hyaluronic acid into the mold, a similar paste also containing hyaluronic acid but lacking the trypan blue was cast on top of the originally placed paste in the mold. Heating was subsequently conducted for 2.5 hours at 45 °C.

A microneedle array having the trypan blue carried preferentially by the distal portions of the microneedles resulted. Figure 9 shows an image of a microneedle array having trypan blue carried by the distal portions of the microneedles.

**Example 5:** A microneedle array substituting fluorescein isothiocyanate (FITC)/human serum albumin (HSA) for trypan blue was formed in a similar manner to that of Example 4.

**Example 6:** A microneedle array substituting botulinium toxin type A (BoNT/A) for trypan blue was also formed in a similar manner to that of Example 4.

**Example 7:** The mechanical properties of the microneedle array of Example 1 were measured using a Stable Microsystem texture analyzer. In performing these measurements, the microneedle array was place on a measuring surface with the microneedles facing upward. The probe from the texture analyzer was contacted with the microneedles and moved axially with respect to the microneedles. The force response as a function of the probe travelling distance was then recorded. Figure 10 shows a plot of force response versus probe travelling distance for the hyaluronic acid microneedle array in comparison to a commercially available microneedle array obtained from Shiseido. As shown in Figure 10, the hyaluronic acid microneedle array had a much higher mechanical strength.

**Example 8:** Skin perforation tests were conducted using human cadaver skin and the microneedle array of Example 5. The microneedle array was placed on the skin with the microneedles facing downward, and pressure was manually applied with a 2 kg weight for approximately one minute. A 190 g weight was then placed on an applicator on the microneedle array, and the weight was held in place for 60 minutes. The weight and applicator were then removed by pulling outward with respect to the skin. Confocal image analyses of the microneedles remaining in the skin were then conducted. The confocal image analyses showed penetration to a depth of about 100 $\mu$m within the skin, which may be suitable for some superficial applications. Figure 11A shows a photograph of the skin sample treated with a dye-labeled microneedle array. It can be seen that the microneedles are uniformly distributed in the skin sample. Figure 11B shows a micrograph of a microneedle array taken prior to penetration of the skin sample, and Figure 11C shows a micrograph of the microneedle array of Figure 11B taken after 5 minutes of penetration of the skin sample. It can be seen that the microneedles have begun to dissolve in the skin.

Skin permeation studies were also conducted using a Franz-Cell Assay. The skin penetration studies were

performed using an immunoglobulin G (IgG) loaded hyaluronic microneedle patch prepared in a manner similar to those described in Examples 1-6. The patch contained 2.8 ± 0.24) μg IgG/patch.

A Franz cell (Logan Instruments) was pretreated with 5 mL of 5% bovine serum albumin (BSA) vehicle in PBS overnight with magnetic stirring at room temperature. The following day, the 5 mL vehicle was replaced with 5 mL of 1% BSA in PBS containing 1x proteinase inhibitor and prewarmed to 32 °C. Human cadaver skin was thawed with room temperature water for 1 hour, cut into pieces to fit one microneedle patch, and then fastened onto a plastic foam with pins. The skin was stretched to make a tight, flat surface and wiped to remove the water on the skin. The patch was applied to the skin using an applicator for 1 minute, and then a weight of 300 g was placed on the patch and left for 4 minutes (5 minute study). A second skin sample was likewise left for 30 minutes. The skin samples were applied onto the Franz cells with the stratum corneum layer facing upwards. The 1% BSA vehicle was then allowed to diffuse through the sample and 0.4 mL aliquots were taken from the receptor arm of the Franz cell and selected intervals. Each time an aliquot was taken 0.4 mL fresh 1% BSA vehicle containing 1x proteinase inhibitor was added to the cell.

The aliquots were then collected and studied using enzyme-linked immunosorbent assay (ELISA). To recover residual IgG from the skin samples, each skin sample was weighed and cut into small pieces. The pieces were incubated at 4 °C for at least 4 hours in a suspension of 50 mg skin per mL of 1% BSA (in PBS) containing 1× proteinase inhibitor. The tissues were then homogenized and rotated with a rocker at 5 °C overnight and subsequently centrifuged at 4700 × g and 5 °C for 15 minutes to collect the supernatant. The supernatant was then analyzed using ELISA. The time dependent results of IgG permeation through the skin samples is provided in Figure 12. This About 14-17 ng of IgG permeated through human cadaver skin after 70 hrs.

**Example 9:** A Toxin (900 kDa)-loaded HA microneedle patch was prepared using BoNT/A (900 kDa) having a concentration of 0.46 mg/ml (potency 4.70E+7 (U/mg)); 160 kDa hyaluronic acid; and a buffer of 20 mM histidine 6.0. 36 μL of toxin solution were added to 100 ml of 20 mM histidine buffer. The concentration after dilution was 0.000166 μg/μl. HA-toxin gel (12 wt. %) was then prepared by adding 109 mg HA fiber (160 kDa) to a 5 ml Norm-Ject HSW syringe. 803.14 mg of toxin solution were added to a second 5ml Norm-Ject HSW syringe. The two syringes were then connected using a female-to-female syringe connector, and the toxin solution was gently injected into the syringe with the HA fiber. The mixture was mixed back and forth for 10 cycles, and this mixing was repeated every five minutes for a total of 7 times. A second gel was prepared to make a backing layer (base). 400 mg of 160 kDa HA was mixed with 1000 mg of pH 6.0 20 mM histidine buffer. The final HA concentration was 28.57 wt. %.

Toxin-loaded microneedle array patches were then prepared. 18.2 mg of HA-toxin gel (12 wt. % HA; toxin conc. 0.1458 ng/mg) were weighed onto a silicone microneedle mold. 125 mg of HA gel (28.57 wt. % HA in 20 mM histidine buffer) was separately weighed out and pressed to a wet paste using two Teflon-sheets. The HA-paste was then cast onto the Ha-toxin solution which was on the silicone microneedle mold. A second Toxin (150 kDa)-loaded HA microneedle patch was prepared similar to procedure as described as above. The Toxin (900 kDa)-loaded HA microneedle and toxin (150 kDa)-loaded microneedle patch were further analyzed by mass recovery, cell-based potency assay (CBPA), and light chain (LC) activity assay.

Mass recovery was determined using ELISA assay with F12-3-8 monoclonal antibodies as the capture antibody, polyclonal detection antibody as the detection antibody. A total of five patches were analyzed, and toxin mass recovery was found to be 80 ± 11.9) % of the targeted loading amount.

CBPA studies were also performed to assess the potency of the 150/ 900 kDa BoNT/A complex in the microneedle arrays described herein.

**Differentiation:** The neuroblastoma cells were cultured for approximately 72 hours in the presence of trisialoganglioside and neuronal supplements to increase sensitivity of the cells to neurotoxin uptake.

**Drug Treatment:** The cells were incubated with the drug for 24 hours during which time the neurotoxin would bind to the cell surface receptor, was internalized, and the light chain endopeptidase domain was translocated into the cytosol where it cleaved $SNAP25_{206}$ between amino acids 197 and 198.

**Accumulation of cleaved $SNAP25_{197}$:** The cells were incubated for another 72 hours to allow for $SNAP2_{197}$ accumulation.

**Quantification of $SNAP2_{197}$ by ElectroChemiluminescence (ECL)-ELISA:** Cell lysates were collected and $SNAP2_{197}$ quantified with the SNAP25 ECL-ELISA. The ECL-ELISA signal for the reference standard, in relative light units, was plotted against the treatment concentration and the potency of the test sample is extrapolated from the standard curve equation.

In the above procedure the reference standard is Botox standard 016 (3 U/mL-0.0938 U/mL). The experimental control was a DS2 900 kDa drug substance lot used to make patches (2 U/mL). The results of this study showed the average recovery to be 69.3 ± 5.96) %.

**Example 10:** Evaluation of 900 kDa BoNT/A toxin loaded hyaluronic acid patches was also completed with the Light-Chain Activity High-Performance Liquid Chromatography (LCA-HPLC) assay. These studies were performed to determine the recovery of 900 kDa BoNT/A toxin from dissolved hyaluronic acid patches using a light chain activity HPLC assay described below.

Four patches were evaluated. Each patch was placed in a 5 mL Eppendorf Protein LoBind tube. Four mL volumes of Digestion Buffer (0.5 mM Zinc Acetate, 0.05% Tween 20, 2 mM DTT in 50 mM HEPES, pH 7.4) were added to each tube. Patches were dissolved at ambient room temperature for 1.5 hours. Tubes were placed on a shaker (200 rpm) throughout the 1.5 hour time period. Triplicate 350 μL volumes from each patch dissolution tube were transferred to 0.6 mL Axygen tubes for testing.

Standard curve concentrations of 0.05, 0.1, 0.5 and 1 ng/mL were prepared using the same material of 900 kDa BoNT/A toxin that was used to prepare the HA patches. The standard curve was prepared in Digestion Buffer. Triplicate 350 μL volumes of each standard curve concentration were transferred to 0.6 mL Axygen tubes for testing.

Samples were incubated for 30 minutes at 37 °C to facilitate sample reduction. Fifty μL volumes of SNAPtide substrate were added to each sample tube. Sample tubes were incubated at ambient room temperature for 72 hours to allow for substrate cleavage. After 72 hours, 25 μL volumes of 5% trifuoracetic acid (TFA) were added to each sample tube to stop substrate cleavage. The contents of each tube were then transferred to HPLC vials for analysis. The fluorescently labeled cleavage product(s) were separated and detected via a RP-HPLC method using a Waters 2695 XE Separations Module (Waters Symmetry300 C18, 3.5pm, 4.6 × 150 mm column) and a Waters 2475 Multi λ Fluorescence Detector. Data were collected and analyzed via Waters Empower Pro software. A standard curve was constructed by plotting the BoNT/A standard curve concentrations (x axis) versus the BoNT/A cleavage product peak areas (y axis). Patch concentrations were extrapolated from the curve. The concentration of BoNT/A contained in each patch was determined by multiplying the BoNT/A patch concentration by 4 (dilution factor).

The resultant test data are presented in Table 1. The average toxin recovery was 2.48 ± 0.77 ng/patch. The average toxin patch potency equates to 116.6 units per patch.

**Table 1: Summary of Light-Chain Activity High-Performance Liquid Chromatography (LCA-HPLC) Assay Results When Evaluating 900 kDa BoNT/A Toxin Loaded Patches**

| | Average Peak Area | Standard Deviation | ng/mL | ng/patch |
|---|---|---|---|---|
| Patch 1 | 914368.3 | 32738.9 | 0.45 | 1.80 |
| Patch 2 | 1793514.7 | 16122.0 | 0.89 | 3.55 |
| Patch 3 | 1249298.0 | 42165.4 | 0.62 | 2.47 |
| Patch 4 | 1059296.3 | 167983.4 | 0.52 | 2.09 |

**Example 11:** A microneedle array with a drug-layer tip was manufactured with a trypan blue (MW = 873 g/mol) to show the distribution of the drug-carrying shell layers on the surface of the microneedles. A first polymer solution (10 wt. % HA) was prepared by first adding 90 mg of HA (MW 160 kDa) to a 5 ml syringe. To another 5-ml syringe 193 μl of 0.4 % trypan blue solution and 616 μl of 20 mM, pH 6.0 histidine buffer were added. The two syringes were connected via a female-to-female connector and mixed thoroughly for a duration of 1 hr. The resulting viscous solution was collected to one syringe, capped and then centrifuged at 3000 rpm for 5 minutes.

A second polymer solution was prepared (33.3 wt. % HA) by adding 1.0 g of 160 kDa HA fiber to a 5 ml syringe, and 2.0 gm of pH 6.0 ml of 20 mM, pH 6.0 histidine buffe to another 5 ml syringe. The two syringes were connected via a syringe connector. The buffer solution was pushed to the syringe containing HA fiber, and the HA fiber was left to absorb the buffer for 2 hours, and then mixed by pushing the syringes back and forth for 10 cycles. The resulting highly viscous HA paste was collected to one syringe, capped and then centrifuged at 4000 rpm for 30 minutes.

A trypan-blue-loaded HA microneedle array was then prepared by casting 9 mg of the first HA solution containing trypan blue onto a microneedle mold. Next, 50 mg of the 33.3 wt. % HA was placed on a round Teflon sheet with diameter of 2 cm, placed another Teflon sheet on the top of HA gel. The sheets were then sandwiched between two pieces of glass slide, and compressed to form a wet HA film. One of the Teflon films was then removed to expose the compressed, wet HA film. The sheet having the HA film thereon was then flipped to place the HA film onto the HA/trypan blue solution in the microneedle mold.

The solutions in the mold were then subjected to a compression step conducted under a compression parameter with a force of 45 kg, and a rate of 0.1 mm/sec, after which the pressure was maintained for a holding time of 60 seconds. After the compression was completed, the Teflon film was peeled using tweezers, and the HA microneedle patch was left to dry at room temperature for 3 hrs.

The trypan-blue loaded microneedle array was then examined under light microscope, and micrographs are shown in Figures 13A and 13B. The microneedles showed blue shell layers 304 at the tips and extending down along the surface of the microneedle bodies 302 (Figure 13A, which includes edge and contour lines to facilitate visualization of the microneedle body 302 and the drug-carrying shell layer 304 of the array 306). A cross-section of the needles (Figure 13B) showed that only the surface or shell layer 304 was blue, while the core or microneedle body 302 was not.

This indicates that only the microneedle surface was covered by trypan blue and that the dye did not diffuse into the microneedle body 302 during the casting process. These results indicate that toxin will be coated on the HA microneedle surface provided that similar procedure will be used.

Referring to Figure 13C, a second microneedle array 330 was manufactured in a similar manner except the HA had a molecular weight of 500 kDa and the concentration was at 16 wt. %. A micrograph of the microneedle array 330 produced with this method can be seen in Figure 13C, showing the shell layer 334 formed on the tip and surface of the microneedle bodies 332.

Preparation of a toxin-loaded HA microneedle with toxin loaded on HA microneedle surface using combination of vacuum and compression molding techniques. A toxin solution with a concentration of 0.46 mg/ml BoNT/A (MW = 900 kDa) and potency of $4.70 \times 10^7$ U/mg was prepared. The microneedle patch was designed to have a target loading of 100 units/patch. To a container containing 100 ml of 20 mM, pH 6.0 histidine buffer, 36 μL of toxin solution were added. The concentration of the toxin after dilution was 0.000166 μg/μl.

In a biosafety hood, a HA-toxin polymer solution (1 wt. % HA) was then prepared by adding 8.8 mg HA fiber (160 kDa) to a 5 ml Norm-Ject HSW syringe. To another 5 ml Norm-Ject HSW syringe, 192.72 mg of toxin solution (0.000166 μg/μl) and an additional 616 mg of 20 mM, pH 6.0 histidine buffer were added. The two syringes were connected using a female-to-female syringe connector and the plunger of the toxin-containing syringe was gently pushed to pass toxin solution into the syringe containing HA fiber. The mixture was gently passed back and forth 10 cycles, and this mixing process was repeated every 5 minutes for a total of 7 times. The mixture was then collected into a single syringe, which was kept at 5 °C overnight.

A second HA polymer solution for forming HA a microneedle backing layer was prepared. 400 mg of 160 kDa HA was mixed with 800 mg of pH 6.0, 20 mM histidine buffer. The final HA concentration of the HA solution was 33.3 wt. %.

9 μl of the HA/toxin solution was pipetted onto a microneedle mold. A vacuum was then applied to allow the HA/toxin solution to fill in the cavities of the microneedle mold. 50 mg of 33.3 wt. % HA was then applied to the mold and compressed at a force of 45 kg, rate of 0.1 mm/sec, and held for 30 seconds. The mold was then left to dry at room temperature under vacuum in the biosafety hood for 3 hrs.

Additional microneedle arrays were produced in a similar manner as that described above. The details of these arrays are summarized in Table 2 below. In these arrays, both 900 kDa toxin and 150 kDa toxin were successfully loaded to into a HA microneedle array. Toxin was loaded into microneedles with different designs, e.g., needle length and needle density. The resulting toxin-loaded HA microneedle arrays were further subjected to mass recovery by ELISA, CBPA (cell-based potency assay), and LC (light chain) activity assay to determine the toxin activity, all of which indicated successful loading as indicated in Table 2 below.

**Table 2: Characterization of Toxin Loaded Microneedle Arrays of Example 11**

| Patch Design | BoNT (kDa) | Target Load | ELISA (Units ± SD) * | LC/A (Units ± SD) | CBPA (Units ± SD) |
|---|---|---|---|---|---|
| 600 μm (length)<br>400 needles/cm$^2$ (density) | 900 | 125 U | 100 ± 14.9 | 116 ± 36 | 86.6 ± 8.3 |
| 600 μm (length)<br>400 needles/cm$^2$ (density) | 150 | 125 U | 74.1 ± 12.5 | 80 ± 7 | 95.4 ± 19.5 |
| 600 μm (length)<br>400 needles/cm$^2$ (density) | 900 | 100 U | 78.9 ± 15.2 | 105.9 ± 17.7 | 57.0 ± 16.8 |
| 700 μm (length)<br>300 needles/cm$^2$ (density) | 900 | 100 U | 108.8 ± 5.9 | 82.2 ± 7.5 | 84.0 ± 7.4 |

**Example 12:** Alternative casting method: Compression vs. Centrifugation. HA microneedle array was manufactured similar to the manner described in Example 11, but using centrifugation process instead of compression, however, it was found that centrifugation requires the solution to have low viscosity in order to fill the solution into a microneedle mold cavity. HA with certain molecular weight (e.g., greater than 10,000 Da) is very viscous even at low concentration (e.g., 1 wt. %). In order to form a microneedle array, multiple centrifugation steps are needed. The process is tedious, less efficient, and has a high probability for contamination and spills, especially when toxin is present.

Thus, HA microneedle arrays cannot be manufactured by centrifugation process at a speeds of 4000 rpm and centrifugation time up to 30 min if the HA concentration is above 5 wt. %. If the HA concentration is significantly reduced to less than 1 wt. %, HA microneedle arrays can be fabricated using centrifugation, but multiple centrifugations must be conducted in order to form robust HA microneedles, otherwise, the microneedles either have defects or have a very thin backing layer leading to a weak microneedle array.

**Example 13:** Rat Digit Abduction Score Studies (DAS). Patches containing HA and toxin were manufactured according to the method described in Example 9 with a size of 1.13 $cm^2$, a microneedle density of 300 microneedles/$cm^2$, and a needle length of 700 μm. Four patches were prepared with respective toxin loads of 100 units/patch, 30 units/patch, 10 units/patch, and 3 units per/patch. The patches were then used in DAS studies as described below.

The potency of a neurotoxin preparation can be routinely assessed by using the Digit Abduction Score (DAS) assay, which measures the local muscle weakening efficacy of a neurotoxin following injection into an animal hindlimb muscle. In the Rat DAS, the animal is a rat. Digit abduction for the rat is scored on a five-point scale with 0 being normal and 4 being maximum reduction in digit abduction. In a rat abduction assay, a DAS score of "1" is typically assigned when loss of abduction is observed with a single digit on the treated limb. A DAS score of "2" is given with a loss of three digits on the treated limb, and a score of "3" is given with loss of abduction in four digits on the treated limb. Finally, rats receive a score of "4" when all five digits of the treated limb have a loss of abduction.

In the present study, all DAS assays were performed in a Class II Type A2 biosafety cabinet (BSC) on 6 rats for each microneedle array patch. On "day 0," the rats were anesthetized with a ketamine/xylazine cocktail (75 mg/kg ketamine, 10 mg/kg xylazine). Once sufficiently anesthetized, a toxin-loaded microneedle patch was applied on the skin over the right tibialis anterior (TA) muscle of each rat. Next, administration was carried out using a high impact spring applicator (Micropoint Technologies, Singapore) to ensure microneedle penetration into the skin. The applicator was held in place, with minimal pressure, on the applied patch from one to five minutes. After the application period was complete, the patch was removed and stored appropriately or bleached.

DAS scores are recorded beginning on Day 1 and on subsequent days. As shown in Figures 14A to 14C, the 100U patch application led to a saturation of response. Additionally, all the rats in that group exhibited the early signs of systemic toxicity. The 30U patch also had a score of 4. The 10 U patch applications led to a DAS score of about 2. This score persisted until about day 10, at which point the DAS score gradually began to decrease. The 3U patch had a score between 1 and 2. Thus, the results clearly demonstrated that toxin-loaded HA microneedle patches can deliver functional toxin in a controlled dosage and release.

**Example 14:** A microneedle array was manufactured following the steps of Example 9, with the exception that the toxin-containing polymer solution contained 0.1 to 2 wt. % of a hyaluronic acid having a molecular weight greater than $3\times10^6$ Da and the polymer solution used to form the base layer and microneedle elongate body contained 5 to 40 wt. % of a hyaluronic acid having a molecular weight in the range of 50,000 Da to about $1\times10^6$ Da. The resulting microneedle array showed the elongate body having a dissolution rate that was much faster than that of the toxin-containing shell layer. The toxin-containing outer layer can thus be designed to have a slower dissolution rate, thereby controlling the release of toxin *in vivo,* and providing a long-acting effect.

Further Considerations

In some embodiments, any of the clauses herein may depend from any one of the independent clauses or any one of the dependent clauses. In one aspect, any of the clauses (e.g., dependent or independent clauses) may be combined with any other one or more clauses (e.g., dependent or independent clauses). In one aspect, a claim may include some or all of the words (e.g., steps, operations, means or components) recited in a clause, a sentence, a phrase or a paragraph. In one aspect, a claim may include some or all of the words recited in one or more clauses, sentences, phrases or paragraphs. In one aspect, some of the words in each of the clauses, sentences, phrases or paragraphs may be removed. In one aspect, additional words or elements may be added to a clause, a sentence, a phrase or a paragraph. In one aspect, the subject technology may be implemented without utilizing some of the components, elements, functions or operations described herein. In one aspect, the subject technology may be implemented utilizing additional components, elements, functions or operations.

The subject technology is illustrated, for example, according to various aspects described below. Various examples of aspects of the subject technology are described as numbered clauses (1, 2, 3, etc.) for convenience. These are provided as examples and do not limit the subj ect technology. It is noted that any of the dependent clauses may be combined in any combination, and placed into a respective independent clause, e.g., clause 1 or clause 20. The other clauses can be presented in a similar manner.

Clause 1. A microneedle array comprising: a base layer; a plurality of microneedles projecting from the base layer, each of the microneedles and the base layer being a single, structurally continuous component comprised of a first hyaluronic acid polymer matrix, each of the plurality of microneedles being an elongate body having a proximal portion continuous with the base layer, the elongate body generally tapering from the proximal portion toward a distal

portion thereof and defining an irregular body geometry; and drug-carrying shell layers at least partially encapsulating and flowed around the elongate bodies of the plurality of microneedles to define an irregular surface boundary against the irregular body geometries thereof, the drug-carrying shell layers each having a predefined outer profile that varies from a respective irregular body geometry of a respective elongate body to permit the array to have a consistent microneedle profile, the drug-carrying shell layers comprising a neurotoxin dispersed in a second hyaluronic acid polymer matrix; wherein the drug-carrying shell layers and the elongate bodies are fused together via an integrated hyaluronic acid polymer matrix comprising an overlap of the first hyaluronic acid polymer matrix and the second hyaluronic acid polymer matrix spanning the irregular surface boundary.

Clause 2. The microneedle array of Clause 1, wherein the first hyaluronic acid polymer matrix has a polymer concentration greater than a polymer concentration of the second hyaluronic acid polymer matrix.

Clause 3. The microneedle array of Clause 1, wherein the first hyaluronic acid polymer matrix has a specific weight greater than a specific weight of the second hyaluronic acid polymer matrix.

Clause 4. The microneedle array of any one of the preceding Clauses, wherein the drug-carrying shell layers cover only the distal portion of the elongate body.

Clause 5. The microneedle array of any one of Clauses 1 to 3, wherein the drug-carrying shell layers completely encapsulate the elongate body of the microneedle.

Clause 6. The microneedle array of any one of the preceding Clauses, wherein the neurotoxin comprises a botulinum toxin.

Clause 7. The microneedle array of Clause 6, wherein the botulinum toxin is selected from the group consisting of Botulinum toxin serotype A (BoNT/A), Botulinum toxin serotype B (BoNT/B), Botulinum toxin serotype C1 (BoNT/C1), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), and mosaic Botulinum toxins and/or variants thereof.

Clause 8. The microneedle array of any one of the preceding Clauses, wherein the microneedle array is configured to deliver about 0.01 to about 100 U/cm2 of the neurotoxin to a patient.

Clause 9. The microneedle array of any one of the preceding Clauses, wherein the microneedle array has a loading concentration of neurotoxin in a range of about 0.01 to about 100,000 U/cm2.

Clause 10. The microneedle array of any one of the preceding Clauses, wherein the second hyaluronic acid polymer matrix comprises about 0.000001% to about 0.01% by weight of the neurotoxin.

Clause 11. The microneedle array of any one of the preceding Clauses, wherein the first hyaluronic acid polymer matrix, the second hyaluronic acid polymer matrix, or both comprises un-crosslinked hyaluronic acid, crosslinked hyaluronic acid, hydrophobically modified hyaluronic acid, or any combination thereof.

Clause 12. The microneedle array of any one of the preceding Clauses, wherein the first hyaluronic acid polymer matrix and the second hyaluronic acid polymer matrix each independently comprises a hyaluronic acid having a molecular weight in a range of about 150 kDa to about 6 MDa.

Clause 13. The microneedle array of any one of the preceding Clauses, wherein the first hyaluronic acid polymer matrix, the second hyaluronic acid polymer matrix, or both further comprises at least one of a glycosaminoglycan, a polysaccharide, collagen, elastin, fibroin, starch, glucomannan, or any combination thereof.

Clause 14. The microneedle array of any one of the preceding Clauses, wherein the proximal portion and the base layer do not comprise an active ingredient.

Clause 15. The microneedle array of any one of the preceding Clauses, wherein the first hyaluronic acid polymer matrix consists of un-crosslinked hyaluronic acid, crosslinked hyaluronic acid, hydrophobically modified hyaluronic acid, or any combination thereof.

Clause 16. The microneedle array of any one of the preceding Clauses, wherein each of the plurality of microneedles has a length ranging between about 25 microns and about 3000 microns.

Clause 17. The microneedle array of any one of the preceding Clauses, wherein each of the plurality of microneedles has substantially the same length.

Clause 18. The microneedle array of any one of the preceding Clauses, wherein each of the plurality of microneedles has a conical or pyramidal geometry.

Clause 19. The microneedle array of any one of the preceding Clauses, wherein each of the plurality of microneedles has a tip width ranging between about 1 micron to about 30 microns.

Clause 20. The microneedle array of any one of the preceding Clauses, wherein a density of microneedles of the microneedle array ranges between about 5 microneedles/cm2 to about 1000 microneedles/cm2.

Clause 21. The microneedle array of any one of the preceding Clauses, wherein the neurotoxin is homogeneously dispersed in the drug-carrying shell layers.

Clause 22. A method for forming a microneedle array, the method comprising: providing a microneedle array mold comprising a plurality of elongate wells; providing a first hyaluronic acid polymer solution comprising a neurotoxin and about 1 wt. % to about 40 wt. % of a hyaluronic acid; dispensing the first hyaluronic acid polymer solution into a

lower portion of each of the elongate wells; providing a second hyaluronic acid polymer solution comprising about 25 wt. % to about 50 wt. % of a hyaluronic acid, wherein a viscosity of the second hyaluronic acid polymer solution is greater than a viscosity of the first hyaluronic acid polymer solution; after dispensing the first hyaluronic acid polymer solution, dispensing the second hyaluronic acid polymer solution into each of the elongate wells, the greater viscosity of the second hyaluronic acid polymer solution causing displacement of at least a portion of the first hyaluronic acid polymer solution from the lower portion of each of the elongate wells to flow around the second hyaluronic acid polymer solution thereby forming a neurotoxin-containing outer layer; and drying the first and second hyaluronic acid polymer solutions in the mold to form a microneedle array comprising a base layer having a plurality of microneedles projecting therefrom, each microneedle comprising an elongate body and the neurotoxin-containing outer layer.

Clause 23. The method of Clause 22, wherein after dispensing the first hyaluronic acid polymer solution, each of the elongate wells is only partially filled leaving an upper portion of each of the elongate wells unfilled.

Clause 24. The method of Clause 22, wherein dispensing the second hyaluronic acid polymer solution into each of the elongate wells comprises overfilling the elongate wells with the second hyaluronic acid polymer solution.

Clause 25. The method of any one of Clauses 22 to 24, wherein drying the first and second hyaluronic acid polymer solutions in the mold comprises heating the first and second hyaluronic acid polymer solutions while in the mold.

Clause 26. The method of any one of Clauses 22 to 24, wherein drying the first and second hyaluronic acid polymer solutions in the mold comprises drying the first and second hyaluronic acid polymer solutions while in the mold at room temperature.

Clause 27. The method of any one of Clauses 22 to 26 further comprising, after dispensing the second hyaluronic acid polymer solution, applying a vacuum.

Clause 28. The method of any one of Clauses 22 to 26, wherein dispensing the first hyaluronic acid polymer solution comprises casting the first hyaluronic acid solution onto the mold; and dispensing the second hyaluronic acid polymer solution comprises casting the second hyaluronic acid polymer solution over the first hyaluronic acid solution and applying an external pressure to second hyaluronic acid polymer solution.

Clause 29. The method of Clause 28, wherein applying the pressure further comprises maintaining the pressure for a predetermined amount of time, whereby a portion of hyaluronic acid polymers from each of the first and second hyaluronic acid polymer solutions become integrated at an interface between the first and second hyaluronic polymer solutions.

Clause 30. The method of any one of Clauses 22 to 29, wherein the first hyaluronic acid polymer solution, the second hyaluronic acid polymer solution, or both further comprises an aqueous buffer having a pH in a range of about 6.0 to about 8.0.

Clause 31. The method of Clause 30, wherein the aqueous buffer is a phosphate buffered saline (PBS) or a histidine buffer.

Clause 32. The method of any one of Clauses 22 to 31, wherein at least a portion of the second hyaluronic acid polymer solution is disposed in an overfilled or base portion of the mold, the second hyaluronic acid polymer solution disposed in the overfilled portion of the mold thereby defining the base layer of the microneedle array.

Clause 33. The method of any one of Clauses 22 to 32, wherein the method further comprises separating the microneedle array from the microneedle array mold.

Clause 34. The method of any one of Clauses 22 to 33, wherein the neurotoxin-containing outer layer covers only a distal portion of the elongate body of the microneedles.

Clause 35. The method of any one of Clauses 22 to 33, wherein the neurotoxin-containing outer layer completely encapsulates the elongate body of the microneedles.

Clause 36. The method of any one of Clauses 22 to 35, wherein the neurotoxin comprises a botulinum toxin.

Clause 37. The method of Clause 36, wherein the botulinum toxin is selected from the group consisting of Botulinum toxin serotype A (BoNT/A), Botulinum toxin serotype B (BoNT/B), Botulinum toxin serotype C1 (BoNT/C1), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), and mosaic Botulinum toxins and/or variants thereof.

Clause 38. The method of any one of Clauses 22 to 37, wherein the microneedle array is configured to deliver about 0.01 to about 100 U/cm2 of the neurotoxin to a patient.

Clause 39. The method of any one of Clauses 22 to 38, wherein the microneedle array has a loading concentration of neurotoxin in a range of about 0.01 to about 100,000 U/cm2.

Clause 40. The method of any one of Clauses 22 to 39, wherein the second hyaluronic acid polymer solution comprises about 0.00000001 to about 1.0 mg/mL of the neurotoxin.

Clause 41. The method of any one of Clauses 22 to 40, wherein the hyaluronic acid of the first hyaluronic acid polymer solution, the second hyaluronic acid polymer solution, or both is at least one selected from un-crosslinked hyaluronic acid, crosslinked hyaluronic acid, hydrophobically modified hyaluronic acid, or any combination thereof.

Clause 42. The method of any one of Clauses 22 to 41, wherein the hyaluronic acid of the first hyaluronic acid

polymer solution, the second hyaluronic acid polymer solution, or both each independently has a molecular weight in a range of about 50 kDa to about 6 MDa.

Clause 43. The method of any one of Clauses 22 to 42, wherein the first hyaluronic acid polymer solution, the second hyaluronic acid polymer solution, or both further comprises at least one of a glycosaminoglycan, a polysaccharide, collagen, elastin, fibroin, starch, glucomannan, or any combination thereof.

Clause 44. The method of any one of Clauses 22 to 43, wherein the second hyaluronic acid polymer solution does not comprise an active ingredient.

Clause 45. The method of any one of Clauses 22 to 44, wherein each of the elongate wells has a depth ranging between about 25 microns and about 3000 microns.

Clause 46. The method of any one of Clauses 22 to 45, wherein each of the elongate wells has substantially the same depth.

Clause 47. The method of any one of Clauses 22 to 46, wherein each the elongate wells has a conical or pyramidal geometry.

Clause 48. The method of any one of Clauses 22 to 47, wherein each microneedle has a tip width ranging between about 1 micron to about 30 microns.

Clause 49. The method of any one of Clauses 22 to 48, wherein the microneedle array mold is configured to produce a density of microneedles of the microneedle array ranging between about 5 microneedles/cm2 to about 1000 microneedles/cm2.

Clause 50. The method of any one of Clauses 22 to 49, wherein the neurotoxin is homogeneously dispersed in the neurotoxin-containing outer layer.

Clause 51. A microneedle array produced by any one of Clauses 22 to 50.

Clause 52. A method for treating a patient, the method comprising: providing a microneedle array of any one of Clauses 1 to 21 and 51; and applying the microneedle array to a skin surface of a patient to embed the plurality of microneedles in the skin surface.

Clause 53. The method of Clause 52, wherein at least a portion of the hyaluronic acid in an outer layer dissolves while the microneedles are embedded in the skin surface to release the neurotoxin to the patient.

Clause 54. The method of Clause 52 or 53, wherein the microneedle array is used to treat forehead lines, crow's feet, frown lines, fineline, hyperhidrosis, scarring, psoriasis, or inflammatory dermatosis.

Clause 55. A microneedle array comprising: a base layer; a plurality of microneedles projecting from the base layer, each of the plurality of microneedles being an elongate body having a proximal portion continuous with the base layer, the elongate body generally tapering from the proximal portion toward a distal portion thereof and defining an irregular body geometry; and at least one drug-carrying shell layer at least partially encapsulating and flowed around the elongate bodies of the plurality of microneedles to define an irregular surface boundary against the irregular body geometries thereof, the at least one drug-carrying shell layer having a predefined outer profile that varies from a respective irregular body geometry of a respective elongate body, the at least one drug-carrying shell layer comprising an active ingredient.

Clause 56. The microneedle array of Clause 55, wherein the at least one drug-carrying shell layer and the elongate bodies are fused together.

Clause 57. The microneedle array of any one of Clauses 55 to 56, wherein the active ingredient comprises an antigen, antibody, or toxin.

Clause 58. The microneedle array of any one of Clauses 55 to 57, wherein the active ingredient is a neurotoxin.

Clause 59. The microneedle array of Clause 58, wherein the neurotoxin is a botulinum toxin.

Clause 60. The microneedle array of any one of Clauses 55 to 59, wherein the plurality of microneedles have a conical or pyramidal geometry.

Clause 61. The microneedle array of Clause 60, wherein the conical or pyramidal geometry has a pitch angle associated therewith such that the plurality of microneedles taper to a point or tip.

Clause 62. The microneedle array of any one of Clauses 55 to 61, wherein the base layer, the plurality of microneedles, and the drug-carrying shell layer each comprise at least one polymer.

Clause 63. The microneedle array of Clause 62, wherein the base layer and the plurality of microneedles comprise the same at least one polymer.

Clause 64. The microneedle array of Clause 62, wherein the active ingredient is dispersed in a polymer.

Clause 65. The microneedle array of Clause 62, wherein the at least one polymer is dissolvable.

Clause 66. The microneedle array of Clause 62, wherein the at least one polymer is hyaluronic acid.

Clause 67. The microneedle array of any one of Clauses 55 to 61, wherein the base layer comprises a first polymer, the plurality of microneedles comprise a second polymer, and the drug-carrying shell layer comprises a third polymer.

Clause 68. A microneedle array comprising: a base layer; a plurality of microneedles projecting from the base layer, each of the plurality of microneedles being an elongate body comprising a first polymer and having a proximal

portion continuous with the base layer, the elongate body generally tapering from the proximal portion toward a distal portion thereof and defining an irregular body geometry; and at least one drug-carrying shell layer at least partially encapsulating and flowed around the elongate bodies of the plurality of microneedles to define an irregular surface boundary against the irregular body geometries thereof, the at least one drug-carrying shell layer having a predefined outer profile that varies from a respective irregular body geometry of a respective elongate body, the at least one drug-carrying shell layer comprising an active ingredient dispersed in a second polymer.

Clause 69. The microneedle array of Clause 68, wherein the first polymer dissolves at a slower rate than the second polymer.

Clause 70. The microneedle array of any one of Clauses 68 to 69, wherein the elongate body further comprises an active ingredient.

Clause 71. The microneedle array of any one of Clauses 68 to 70, wherein the second polymer dissolves at a slower rate than the first polymer.

Clause 72. The microneedle array of Clause 71, wherein the second polymer comprises high molecular weight hyaluronic acid at a concentration of about 0.1 to about 2 wt. %.

Clause 73. The microneedle array of Clause 71, wherein the first polymer comprises low molecular weight hyaluronic acid at a concentration of about 5 wt % to about 40 wt %.

Clause 74. A method for forming a microneedle array, the method comprising: providing a microneedle array mold comprising a plurality of elongate wells; providing a first fluid comprising an active ingredient; dispensing the first fluid into a lower portion of each of the elongate wells; providing a second fluid, wherein a viscosity of the second fluid is greater than a viscosity of the first fluid; after dispensing the first fluid, dispensing the second fluid into each of the elongate wells, the greater viscosity of the second fluid causing displacement of at least a portion of the first fluid from the lower portion of each of the elongate wells to flow around the second fluid thereby forming an active ingredient-containing outer layer; and drying the first and second fluids in the mold to form a microneedle array comprising a base layer having a plurality of microneedles projecting therefrom, each microneedle comprising an elongate body and the active ingredient-containing outer layer.

Clause 75. The method of Clause 74, wherein the first fluid comprises a hyaluronic acid polymer solution.

Clause 76. The method of any one of Clauses 74 to 75, wherein the active ingredient comprises a neurotoxin.

Clause 77. The method of any one of Clauses 74 to 76, wherein the second fluid comprises a hyaluronic acid polymer solution consisting essentially of a hyaluronic acid in admixture with a solvent.

Clause 78. The method of any one of Clauses 74 to 77, wherein the second fluid comprises at least one polymer.

Clause 79. The method of Clause 78, wherein the second fluid further comprises one or more additional polymers.

Clause 80. The method of Clause 78, wherein the second fluid further comprises excipients.

Clause 81. A method for forming a microneedle array, the method comprising: providing a microneedle array mold comprising a plurality of elongate wells; providing a first fluid comprising an active ingredient; dispensing the first fluid into a lower portion over the mold; providing a second fluid, wherein a viscosity of the second fluid is greater than a viscosity of the first fluid; after dispensing the first fluid, casting the second fluid over the first fluid; applying a pressure above the second fluid in a first phase of compression, causing the first fluid to flow into the elongate wells and around the second fluid thereby forming an active ingredient-containing shell layer around an elongate body of a microneedle; maintaining the pressure for a period of time in a second phase of compression, creating a matrix that binds the active ingredient-containing shell layer to the elongate body of the microneedle; and drying the first and second fluids in the mold to form a microneedle array comprising a base layer having a plurality of microneedles projecting therefrom, each microneedle comprising an elongate body and the active ingredient-containing shell layer.

Clause 82. The method of Clause 81, wherein the step of casting the second fluid over the first fluid overfills the elongate wells of the mold, forming a single, continuous layer above the elongate wells of the mold.

Clause 83. The method of any one of Clauses 81 to 82, wherein the first fluid and the second fluid comprise a hyaluronic acid polymer solution.

Clause 84. The method of Clause 83, wherein the first fluid comprises less than 5 wt. % of 160 kDA hyaluronic acid.

Clause 85. The method of any one of Clauses 81 to 84, wherein the step of dispensing the first fluid is performed under a vacuum.

Clause 86. A method for forming a microneedle array, the method comprising: providing a microneedle array mold comprising a plurality of elongate wells; providing a first fluid comprising an active ingredient; providing a second fluid, wherein a viscosity of the second fluid is greater than a viscosity of the first fluid; simultaneously injecting the first fluid and the second fluid into the microneedle array mold; compressing the microneedle array mold to form an integral interface between the first fluid and the second fluid; and drying the first and second fluids in the mold to form a microneedle array comprising a base layer having a plurality of microneedles projecting therefrom, each microneedle comprising an elongate body and an active ingredient-containing layer.

Clause 87. The method of Clause 86, wherein the elongate body and the active ingredient-containing layer are structurally integrated with one another.

Clause 88. The method of any one of Clauses 86 to 87, wherein the first fluid and second fluid comprise a polymer.

Clause 89. The method of Clause 88, wherein the elongate body has a greater concentration of polymer than a concentration of polymer of the active ingredient-containing layer.

Clause 90. The method of any one of Clauses 86 to 89, wherein the elongate body has a specific gravity that is greater than a specific gravity of the active ingredient-containing layer.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

There may be many other ways to implement the subject technology. Various functions and elements described herein may be partitioned differently from those shown without departing from the scope of the subject technology. Various modifications to these configurations will be readily apparent to those skilled in the art, and generic principles defined herein may be applied to other configurations. Thus, many changes and modifications may be made to the subject technology, by one having ordinary skill in the art, without departing from the scope of the subject technology.

It is understood that the specific order or hierarchy of steps in the processes disclosed is an illustration of exemplary approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

As used herein, the phrase "at least one of" preceding a series of items, with the term "and" or "or" to separate any of the items, modifies the list as a whole, rather than each member of the list (i.e., each item). The phrase "at least one of" does not require selection of at least one of each item listed; rather, the phrase allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

Terms such as "top," "bottom," "front," "rear" and the like as used in this disclosure should be understood as referring to an arbitrary frame of reference, rather than to the ordinary gravitational frame of reference. Thus, a top surface, a bottom surface, a front surface, and a rear surface may extend upwardly, downwardly, diagonally, or horizontally in a gravitational frame of reference.

Furthermore, to the extent that the term "include," "have," or the like is used in the description or the claims, such term is intended to be inclusive in a manner similar to the term "comprise" as "comprise" is interpreted when employed as a transitional word in a claim.

The word "exemplary" is used herein to mean "serving as an example, instance, or illustration." Any embodiment described herein as "exemplary" is not necessarily to be construed as preferred or advantageous over some embodiments.

A reference to an element in the singular is not intended to mean "one and only one" unless specifically stated, but rather "one or more." Pronouns in the masculine (e.g., his) include the feminine and neuter gender (e.g., her and its) and vice versa. The term "some" refers to one or more. Underlined and/or italicized headings and subheadings are used for convenience only, do not limit the subject technology, and are not referred to in connection with the interpretation of the description of the subject technology. All structural and functional equivalents to the elements of the various configurations described throughout this disclosure that are known or later come to be known to those of ordinary skill in the art are expressly incorporated herein by reference and intended to be encompassed by the subject technology. Moreover, nothing disclosed herein is intended to be dedicated to the public regardless of whether such disclosure is explicitly recited in the above description.

## Claims

**1.** A microneedle array comprising:

a base layer;

a plurality of microneedles projecting from the base layer, each of the plurality of microneedles being an elongate body having a proximal portion continuous with the base layer, the elongate body generally tapering from the proximal portion toward a distal portion thereof and defining an irregular body geometry; and

at least one drug-carrying shell layer at least partially encapsulating and flowed around the elongate bodies of the plurality of microneedles to define an irregular surface boundary against the irregular body geometries

thereof, the at least one drug-carrying shell layer having a predefined outer profile that varies from a respective irregular body geometry of a respective elongate body, the at least one drug-carrying shell layer comprising an active ingredient.

**2.** The microneedle array of Claim 1, wherein the at least one drug-carrying shell layer and the elongate bodies are fused together.

**3.** The microneedle array of Claim 1 or 2, wherein the active ingredient comprises an antigen, antibody, or toxin.

**4.** The microneedle array of any one of Claims 1 to 3, wherein the plurality of microneedles is formed from a first material and the at least one drug-carrying shell layer is formed from a second material, and wherein the irregular surface boundary is formed during casting of the microneedle array by a fluidic interaction between the first and the second materials as the first material, having a higher viscosity than the second material, displaces the second material within an elongate well of a mold in which the second material resides.

**5.** The microneedle array of any one of Claims 1 to 4, wherein the plurality of microneedles are molded from a first material and the at least one drug-carrying shell layer is molded from a second material that has a lower viscosity than the first material and which is displaced by the first material during molding such that when cured, the first and second materials form the irregular surface boundary therebetween.

**6.** The microneedle array of any one of Claims 1 to 5, wherein the base layer, the plurality of microneedles, and the drug-carrying shell layer each comprise at least one polymer.

**7.** The microneedle array of Claim 6, wherein the at least one polymer is hyaluronic acid.

**8.** The microneedle array of any one of Claims 1 to 7, wherein the base layer comprises a first polymer, the plurality of microneedles comprise a second polymer, and the drug-carrying shell layer comprises a third polymer.

**9.** A method for forming a microneedle array, the method comprising:

providing a microneedle array mold comprising a plurality of elongate wells;
providing a first fluid comprising an active ingredient;
dispensing the first fluid into a lower portion of each of the elongate wells;
providing a second fluid, wherein a viscosity of the second fluid is greater than a viscosity of the first fluid;
after dispensing the first fluid, dispensing the second fluid into each of the elongate wells, the greater viscosity of the second fluid causing displacement of at least a portion of the first fluid from the lower portion of each of the elongate wells to flow around the second fluid thereby forming an active ingredient-containing outer layer; and
drying the first and second fluids in the mold to form a microneedle array comprising a base layer having a plurality of microneedles projecting therefrom, each microneedle comprising an elongate body and the active ingredient-containing outer layer.

**10.** The method of Claim 9, wherein the first fluid comprises a hyaluronic acid polymer solution.

**11.** The method of Claim 9 or 10, wherein the active ingredient comprises a neurotoxin.

**12.** The method of any one of Claims 9 to 11, wherein the second fluid comprises a hyaluronic acid polymer solution consisting essentially of a hyaluronic acid in admixture with a solvent.

**13.** The method of any one of Claims 9 to 12, wherein the second fluid comprises at least one polymer.

**14.** The method of Claim 13, wherein the second fluid further comprises one or more additional polymers, or wherein the second fluid further comprises excipients.

**15.** A microneedle array comprising:

a base layer;
a plurality of microneedles projecting from the base layer, each of the microneedles and the base layer being a single, structurally continuous component comprised of a first hyaluronic acid polymer matrix, each of the

plurality of microneedles being an elongate body having a proximal portion continuous with the base layer, the elongate body generally tapering from the proximal portion toward a distal portion thereof and defining an irregular body geometry; and

drug-carrying shell layers at least partially encapsulating and flowed around the elongate bodies of the plurality of microneedles to define an irregular surface boundary against the irregular body geometries thereof, the drug-carrying shell layers each having a predefined outer profile that varies from a respective irregular body geometry of a respective elongate body to permit the array to have a consistent microneedle profile, the drug-carrying shell layers comprising a neurotoxin dispersed in a second hyaluronic acid polymer matrix;

wherein the drug-carrying shell layers and the elongate bodies are fused together via an integrated hyaluronic acid polymer matrix comprising an overlap of the first hyaluronic acid polymer matrix and the second hyaluronic acid polymer matrix spanning the irregular surface boundary.

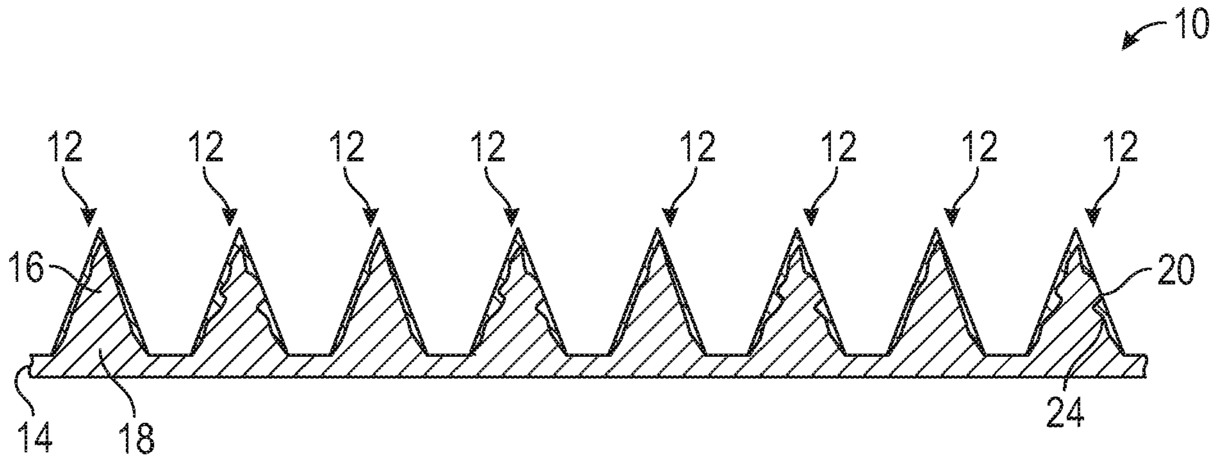


FIG. 1A

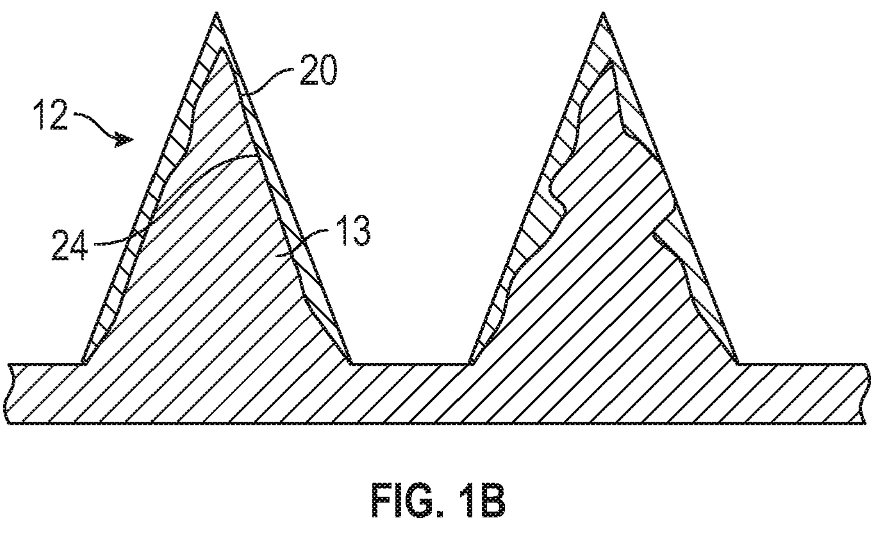


FIG. 1B

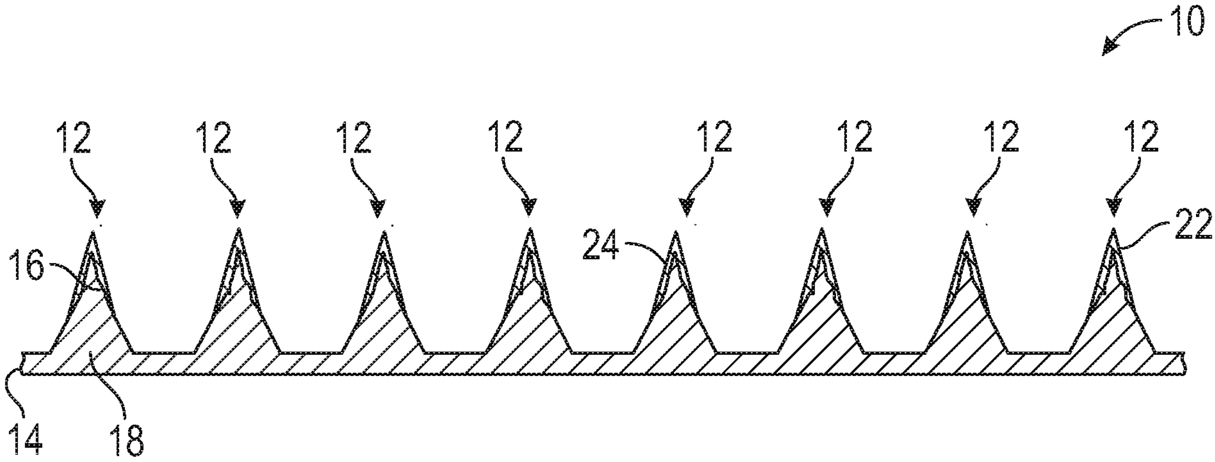


FIG. 2A

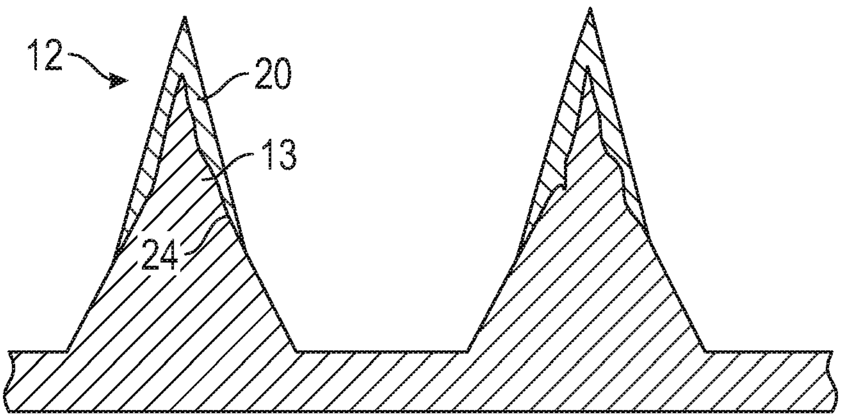


FIG. 2B

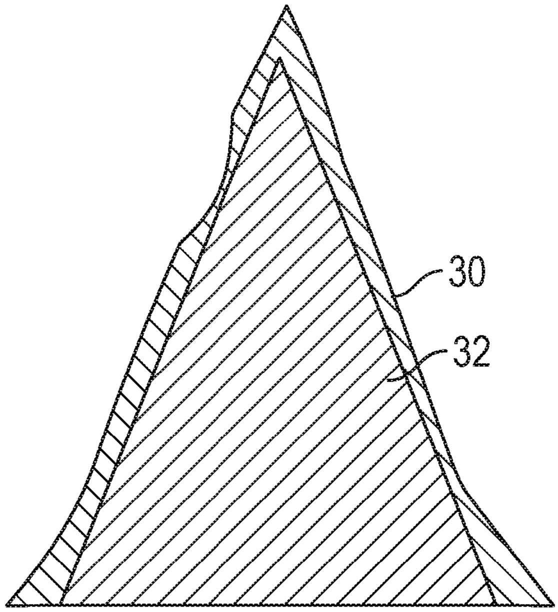


FIG. 3

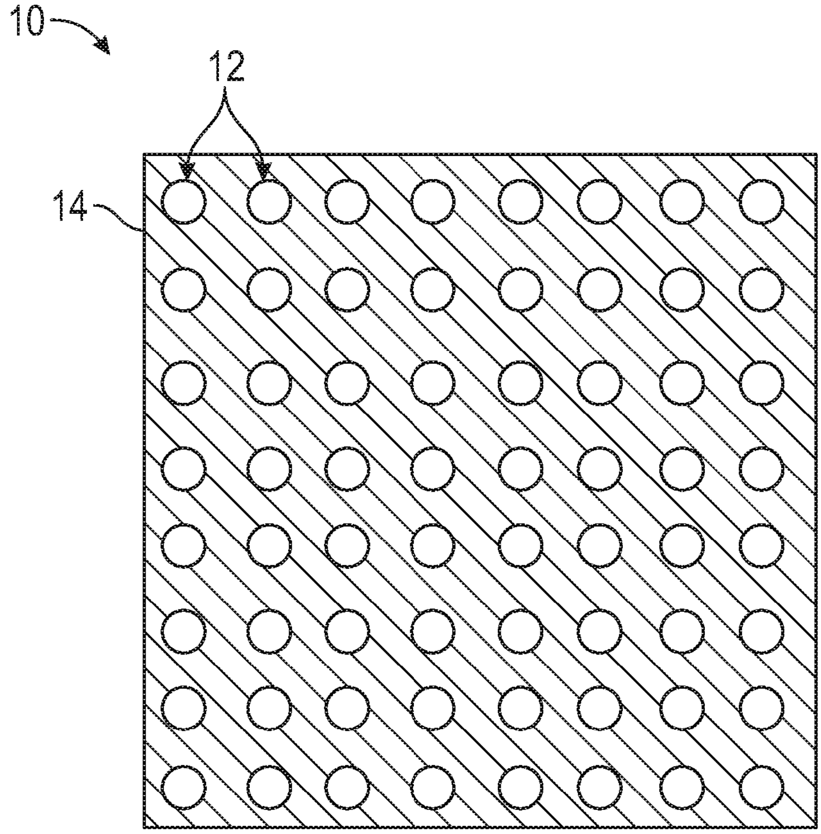


FIG. 4

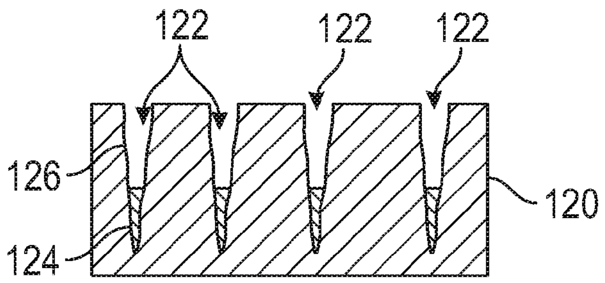


FIG. 5A

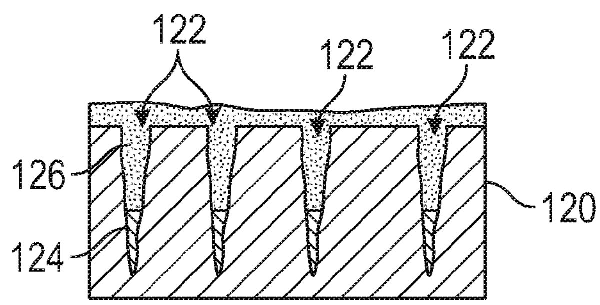


FIG. 5B

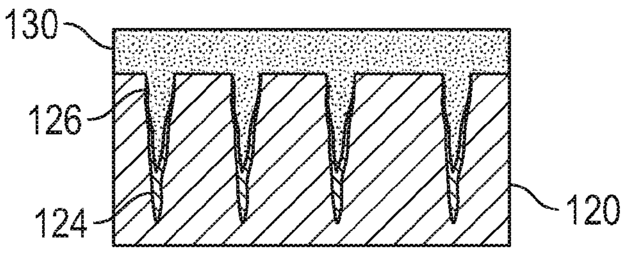


FIG. 5C

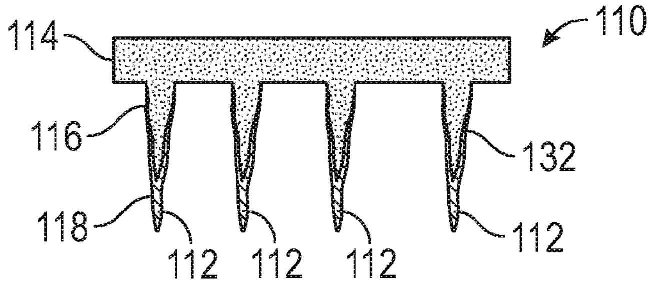


FIG. 5D

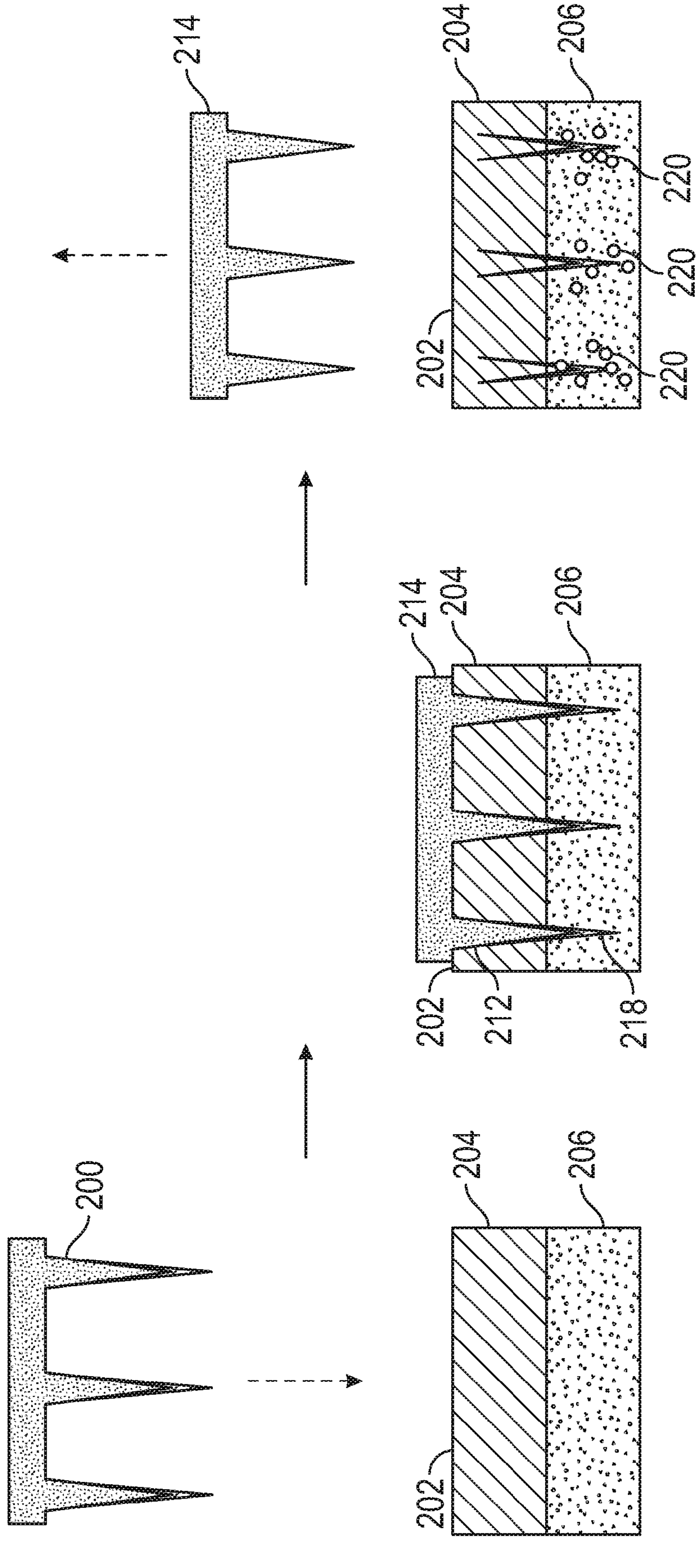


FIG. 6A

FIG. 6B

FIG. 6C

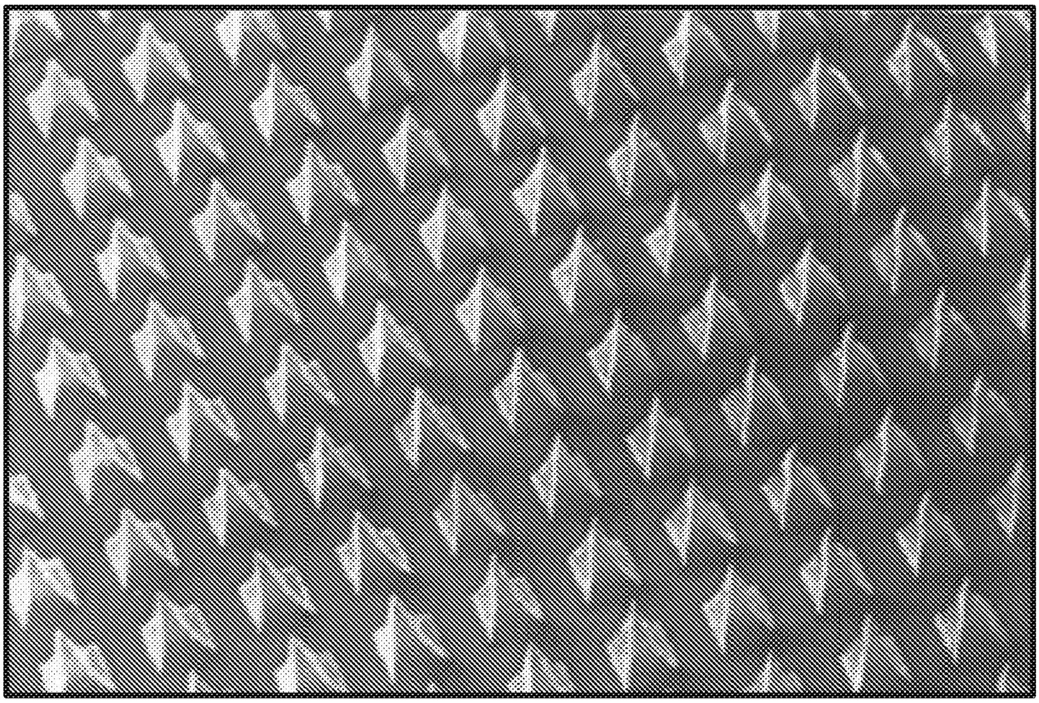

FIG. 7

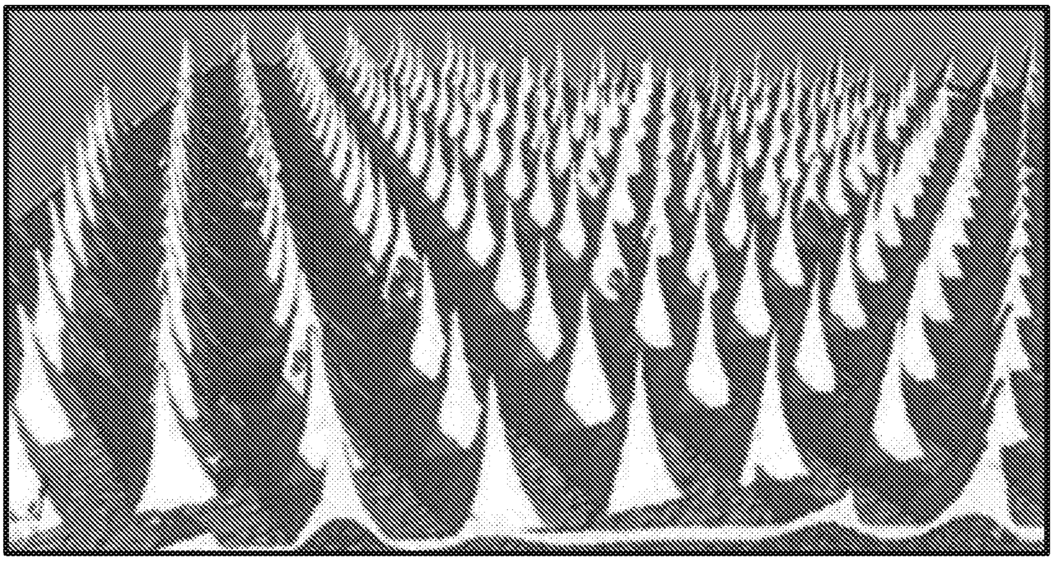

FIG. 8

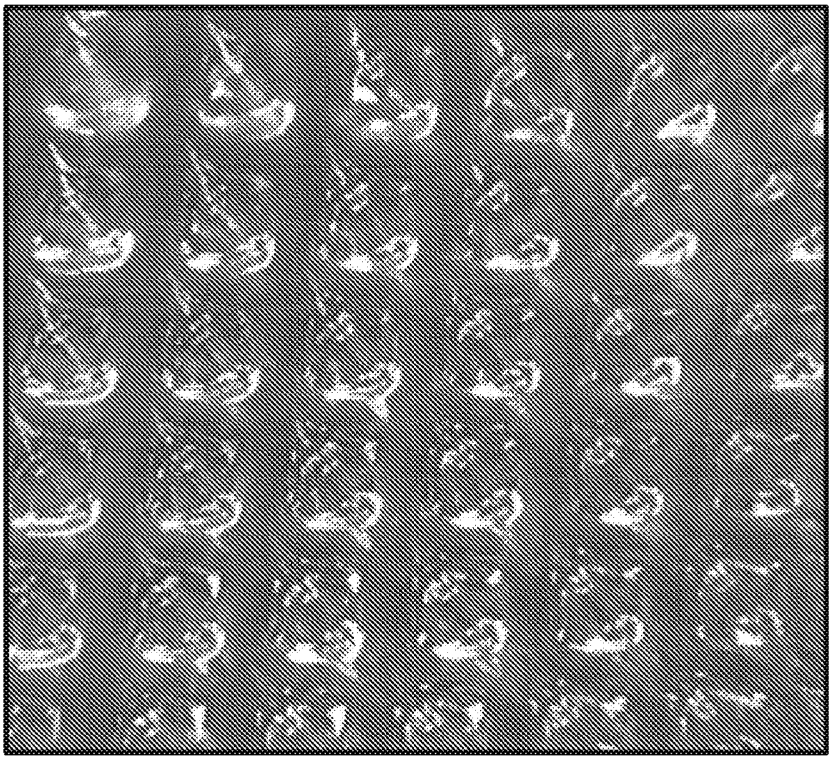

FIG. 9

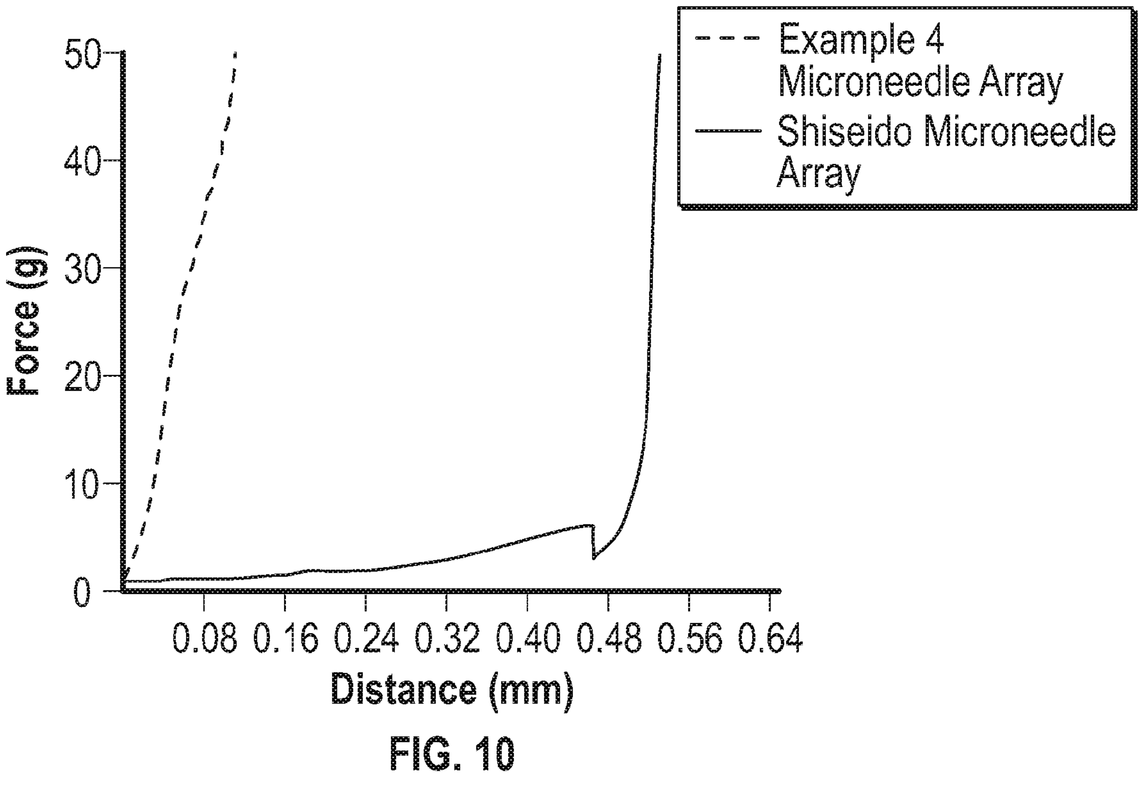
Example 4
Microneedle Array
Shiseido Microneedle
Array
Force (g)
0
10
20
30
40
50
0.08
0.16
0.24
0.32
0.40
0.48
0.56
0.64
Distance (mm)

FIG. 10

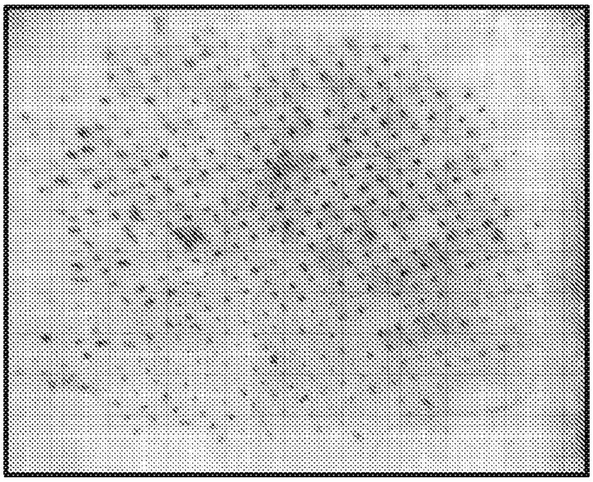

FIG. 11A

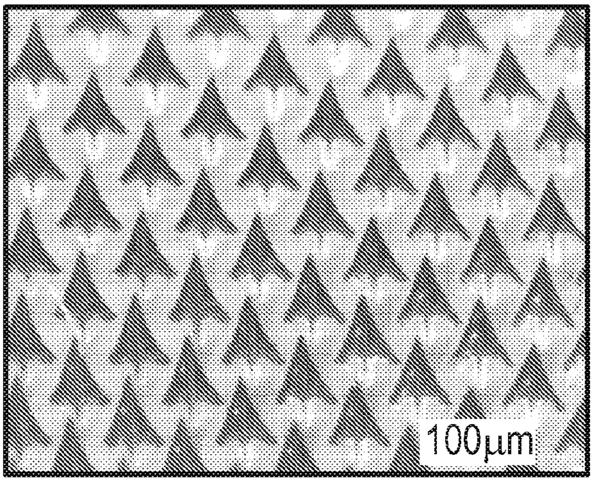


FIG. 11B

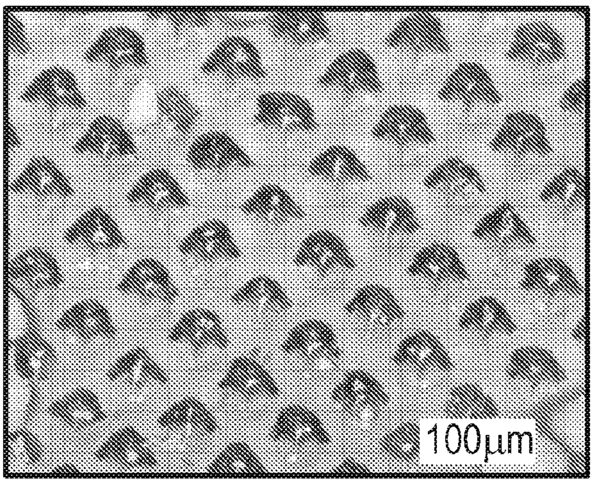


FIG. 11C

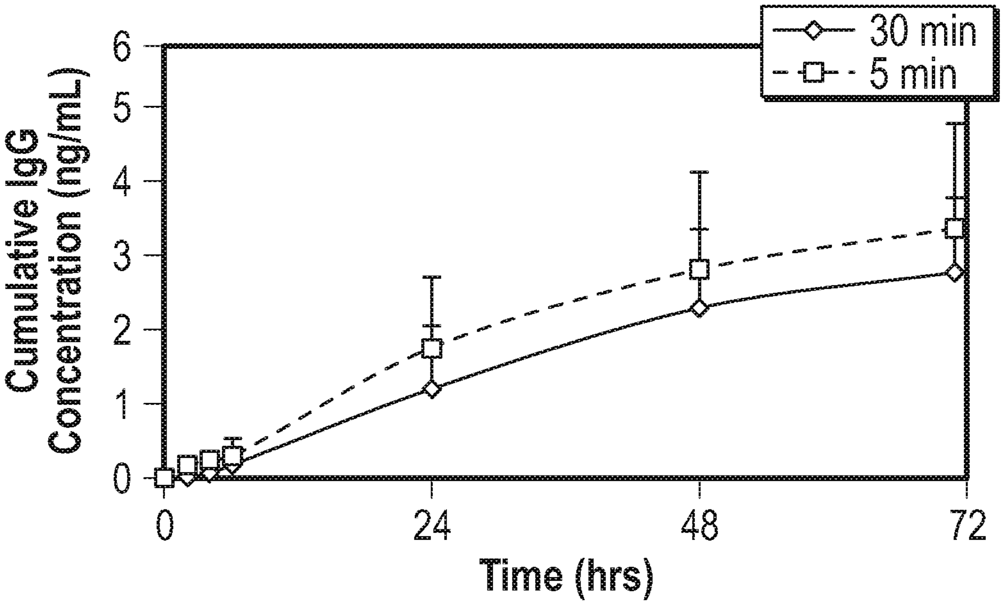


FIG. 12

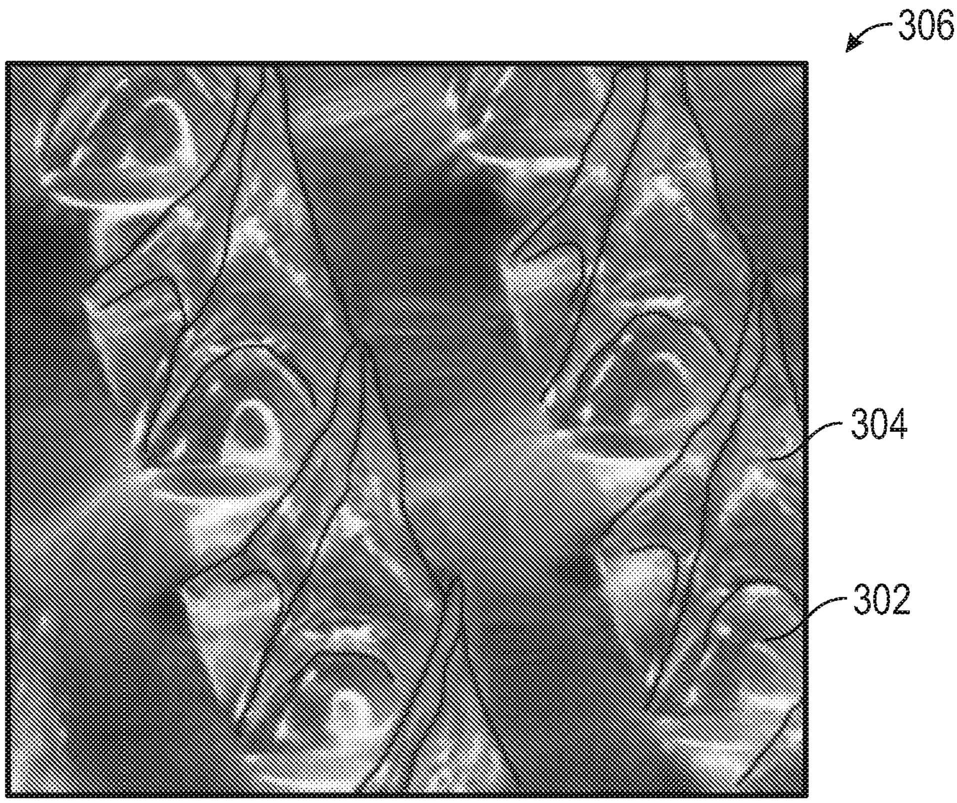


FIG. 13A

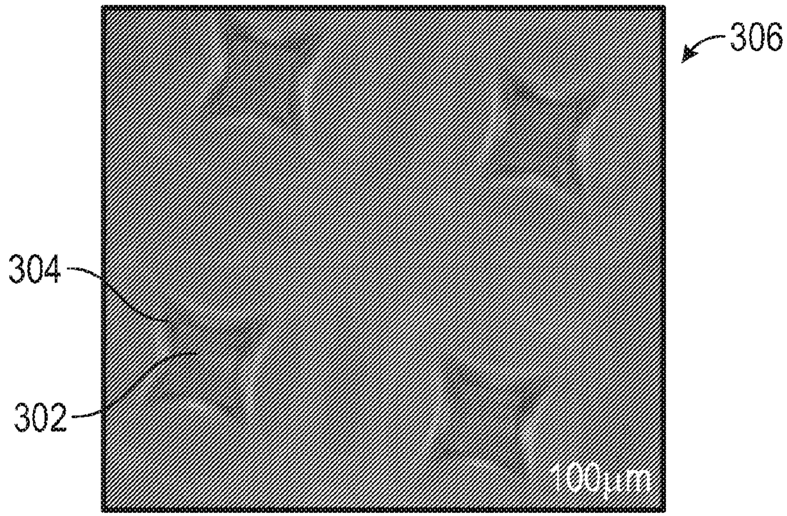


FIG. 13B

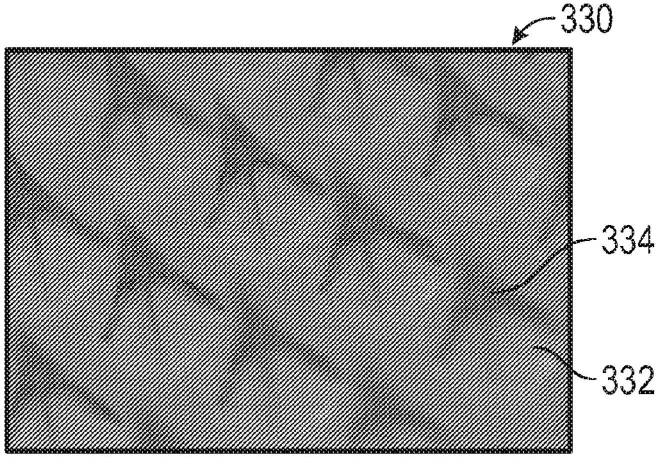


FIG. 13C

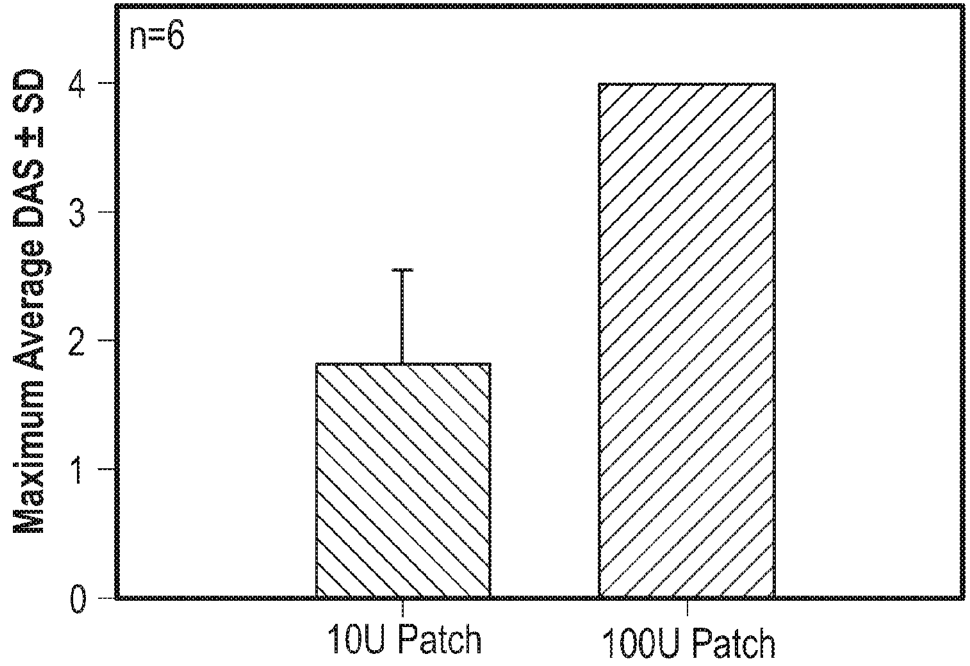


FIG. 14A

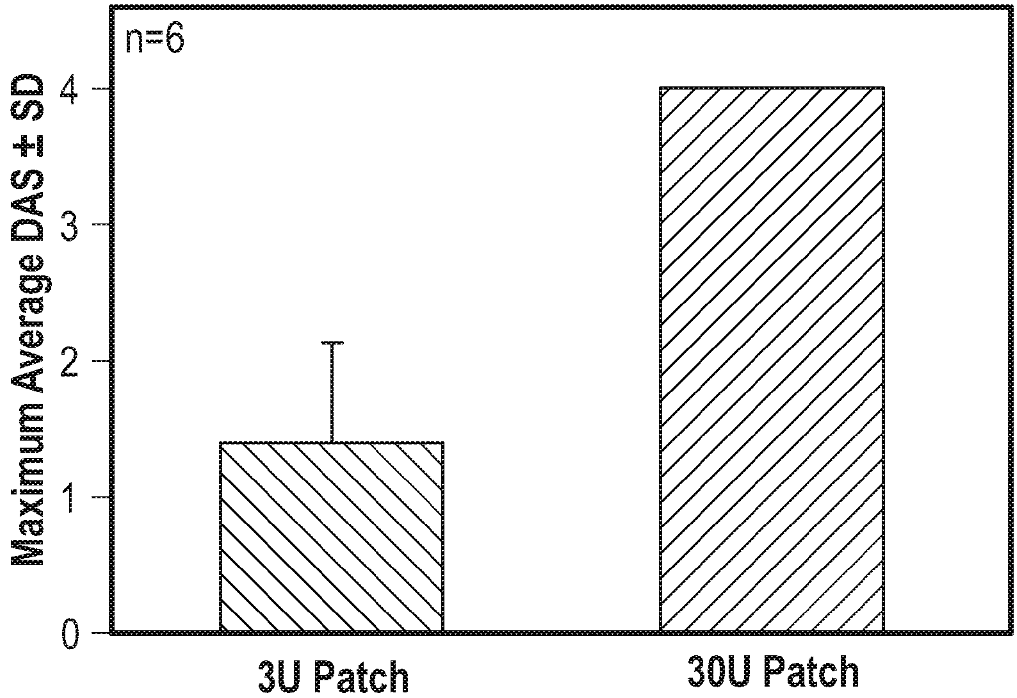


FIG. 14B

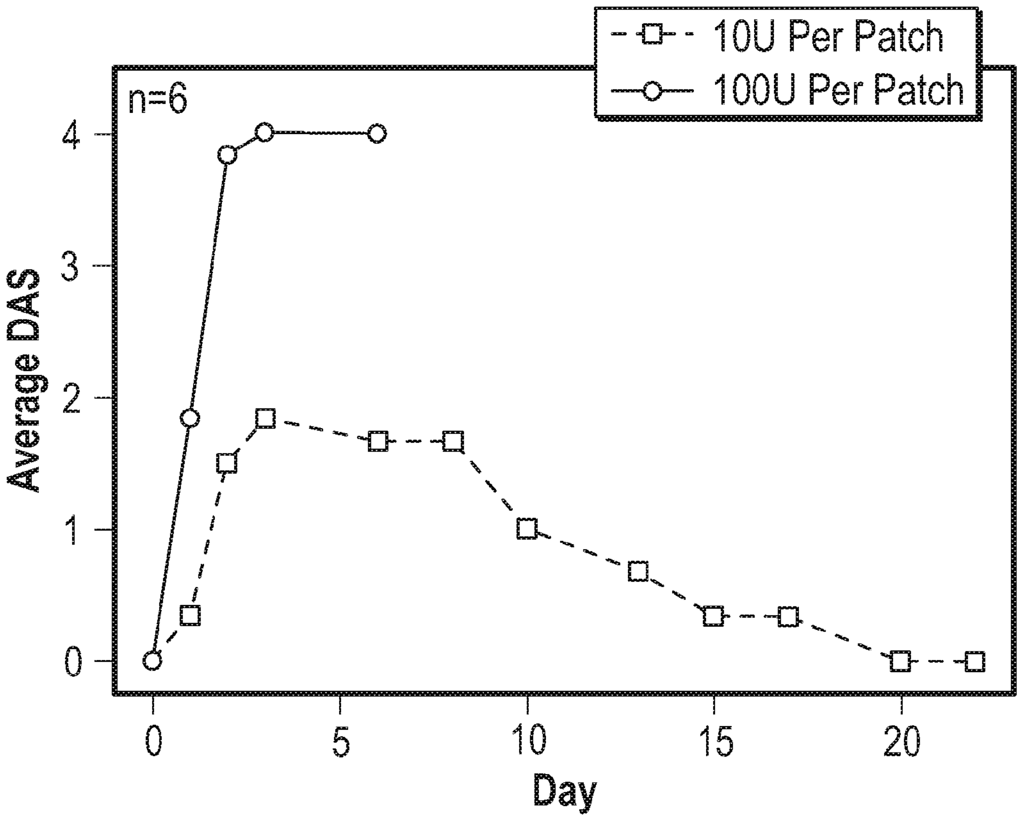


FIG. 14C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62764685
- US 93236518
- US 20160279401 A